# EUROPÄISCHE PATENTSCHRIFT

(11) **EP 2 907 173 B1**
(45) Veröffentlichungstag und Bekanntmachung des Hinweises auf die Patenterteilung: **21.03.2018**
(21) Anmeldenummer: 13763188.3
(22) Anmeldetag: 11.09.2013
(51) Int. Cl.: H01L 51/50, H01L 51/00

(54) **ELEKTRONISCHE VORRICHTUNG**
ELECTRONIC DEVICE
DISPOSITIF ÉLECTRONIQUE

(30) Priorität: 09.10.2012 EP 12006991
(43) Veröffentlichungstag der Anmeldung: 19.08.2015
(73) Patentinhaber: Merck Patent GmbH, 64293 Darmstadt (DE)
(72) Erfinder: VOGES, Frank, 67098 Bad Duerkheim (DE); KROEBER, Jonas Valentin, 60311 Frankfurt am Main (DE); PFLUMM, Christof, 64291 Darmstadt-Arheilgen (DE); KAISER, Joachim, 64289 Darmstadt (DE); BUESING, Arne, 65929 Frankfurt am Main (DE)
(86) Internationale Anmeldenummer: PCT/EP2013/002727
(87) Internationale Veröffentlichungsnummer: WO 2014/056565

(56) Entgegenhaltungen:
- WO-A1-2006/122630
- WO-A1-2013/083216
- WO-A1-2013/135352
- US-A1- 2007 231 596
- US-A1- 2011 215 308

## Beschreibung

Die vorliegende Anmeldung betrifft eine elektronische Vorrichtung enthaltend eine Lochtransportschicht A, eine dotierte Lochtransportschicht B und eine Lochtransportschicht C, wobei die Lochtransportschichten A, B und C zwischen Anode und emittierender Schicht angeordnet sind, und wobei Lochtransportschicht B kathodenseitig von Lochtransportschicht A und Lochtransportschicht C kathodenseitig von Lochtransportschicht B angeordnet ist.

Unter elektronischen Vorrichtungen im Sinne dieser Anmeldung werden insbesondere sogenannte organische elektronische Vorrichtungen verstanden (organic electronic devices), welche organische Halbleitermaterialien als Funktionsmaterialien enthalten. Nochmals insbesondere werden darunter organische Elektrolumineszenzvorrichtungen (OLEDs) und andere elektronische Vorrichtungen verstanden, welche weiter unten aufgeführt sind.

Der Aufbau von OLEDs, in denen organische Halbleiter als funktionelle Materialien eingesetzt werden, ist beispielsweise in US 4539507, US 5151629, EP 0676461 und WO 98/27136 beschrieben.

Bei den betreffenden elektronischen Vorrichtungen, insbesondere OLEDs, besteht großes Interesse an der Verbesserung der Leistungsdaten, insbesondere Lebensdauer, Effizienz und Betriebsspannung.

Die Effizienz und Lebensdauer von elektronischen Vorrichtungen wie OLEDs wird unter anderem durch die Ladungsträgerbalance von Elektronen und Löchern in der Vorrichtung bestimmt. Diese Balance stellt sich durch die Ladungsträgerverteilung und die damit verbundene Feldverteilung in der Vorrichtung ein.

Für gute Leistungsdaten sind insbesondere gute Mobilitäten der Ladungsträger in den Lochtransportschichten und gute Loch-Injektionseigenschaften entscheidend. Weiterhin ist es von entscheidender Bedeutung, dass der Unterschied der HOMOs der Materialien der verschiedenen Lochtransportschichten nicht zu groß ist.

Im Stand der Technik bekannt ist die Verwendung einer p-dotierten Lochtransportschicht, gefolgt von einer nicht dotierten Elektronenblockierschicht, zwischen Anode und emittierender Schicht (WO 2002/041414). Auf die p-dotierte Lochtransportschicht folgt in diesem Fall keine weitere Lochtransportschicht, sondern direkt die emittierende Schicht.

Weiterhin ist im Stand der Technik die Verwendung von zwei oder mehr Lochtransportschichten zwischen Anode und emittierender Schicht bekannt (WO 2010/094378).

Weiterhin offenbart US 2011/0215308 A1 eine OLED enthaltend eine Mehrzahl von Lochtransportschichten, die entweder aus einem Lochtransportmaterial, einem Ladungsträgererzeugungsmaterial (charge generation material), oder einer Mischung beider Materialien bestehen.

Es stellt sich ausgehend von diesem Stand der Technik die technische Aufgabe, elektronische Vorrichtungen, insbesondere OLEDs, bereitzustellen, welche verbesserte Leistungsdaten, insbesondere in Bezug auf Lebensdauer und Effizienz, aufweisen.

Überraschend wurde nun gefunden, dass die Verwendung einer p-dotierten Lochtransportschicht zwischen einer ersten Lochtransportschicht und einer weiteren Lochtransportschicht, gesehen von der Anode, eine Verbesserung in den oben genannten Punkten bewirkt und somit die technische Aufgabe löst.

Gegenstand der vorliegenden Anmeldung ist somit eine elektronische Vorrichtung gemäß Anspruch 1.

Die erfindungsgemäße elektronische Vorrichtung weist den Vorteil auf, dass sie höhere Effizienz, bevorzugt kombiniert mit längerer Lebensdauer aufweist. Weiterhin lässt sie sich bei vergleichsweise niedriger Spannung betreiben.

Die erfindungsgemäße Vorrichtung weist weiterhin den Vorteil auf, dass damit Materialien mit tiefliegendem HOMO in einer Lochtransportschicht verwendet werden können, insbesondere in Kombination mit Materialien mit höherliegendem HOMO in einer anderen Lochtransportschicht.

Dadurch, dass gemäß der Erfindung nur die Lochtransportschichten A' und B p-dotiert sein müssen, sind gegenüber einem Aufbau, bei dem alle Lochtransportschichten p-dotiert sind, die Menge an benötigtem p-Dotanden und damit die Kosten geringer. Dies stellt einen Vorteil gegenüber Vorrichtungen gemäß dem Stand der Technik dar, bei denen alle Lochtransportschichten p-dotiert sind.

Unter einer Lochtransportschicht wird im Rahmen der vorliegenden Anmeldung eine organische Schicht verstanden, welche lochtransportierende Eigenschaften aufweist. Insbesondere wird darunter eine organische Schicht verstanden, die sich zwischen Anode und emittierender Schicht befindet und lochtransportierende Eigenschaften aufweist. Unter einem Lochtransportmaterial wird entsprechend ein Material mit lochtransportierenden Eigenschaften verstanden.

Unter einem p-Dotanden wird eine Verbindung verstanden, welche die andere in der Schicht vorhandene Verbindung (die Matrix) zumindest teilweise oxidieren kann und auf diese Weise die Leitfähigkeit der Schicht erhöht. Typischerweise sind p-Dotanden gemäß der vorliegenden Anmeldung organische Elektronenakzeptorverbindungen.

Eine Matrix bezeichnet dabei diejenige Verbindung bzw. diejenigen Verbindungen, die in einer Schicht enthaltend einen Dotanden die überwiegende Komponente (Gew.-%) darstellen. Entsprechend stellt der Dotand die Mindermengen-Komponente in der entsprechenden Schicht dar. Entsprechendes gilt für die speziellen Bezeichnungen Lochtransportmaterial-Matrix und p-Dotand.

Die erfindungsgemäße elektronische Vorrichtung ist bevorzugt gewählt aus organischen lichtemittierenden Transistoren (OLETs), organischen lichtemittierenden elektrochemischen Zellen (OLECs), organischen Laserdioden (O-Laser) und organischen Elektrolumineszenzvorrichtungen (OLEDs).

Besonders bevorzugt sind organische Elektrolumineszenzvorrichtungen (OLEDs).

Die Anode der elektronischen Vorrichtung besteht bevorzugt aus einem Material mit hoher Austrittsarbeit. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

Gemäß einer bevorzugten Ausführungsform der Erfindung ist die elektronische Vorrichtung dadurch gekennzeichnet, dass die Anode Wolframoxid, Molybdänoxid und/oder Vanadiumoxid enthält.

Bevorzugt ist die oben genannte Anode enthaltend Wolframoxid, Molybdänoxid und/oder Vanadiumoxid derart aufgebaut, dass sie aus Indium-Zinn-Oxid (ITO) besteht, welches mit Wolframoxid, Molybdänoxid und/oder Vanadiumoxid beschichtet ist.

Die Lochtransportschicht A' enthält bevorzugt einen p-Dotanden gewählt aus organischen Elektronenakzeptorverbindungen.

Besonders bevorzugte Ausführungsformen von p-Dotanden sind unten beschrieben im Zusammenhang mit p-Dotanden der Lochtransportschicht B.

Der p-Dotand in Lochtransportschicht A' liegt bevorzugt in einer Konzentration von 0.1 bis 20 Vol-%, bevorzugt 0.5 bis 12 Vol-%, besonders bevorzugt 1 bis 8 Vol-% und ganz besonders bevorzugt 2 bis 6 Vol-% vor.

Die Lochtransportmaterial-Matrix der Lochtransportschicht A' kann ein beliebiges organisches Material mit lochtransportierenden Eigenschaften darstellen.

Bevorzugt sind als Lochtransportmaterial-Matrix für die Lochtransportschicht A' Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Mono-Triarylamine (z. B. gemäß
WO 2012/034627 oder der noch nicht offengelegten EP 12000929.5), Spirobifluoren-Tetrakis-Triarylamine, beispielsweise Spiro-TAD oder Spiro-TTB, Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß WO 2013/083216) und Dihydroacridin-Derivate (z. B. gemäß WO 2012/150001).

Bevorzugt ist die Lochtransportmaterial-Matrix gewählt aus Mono-Triarylaminverbindungen, besonders bevorzugt aus Mono-Triarylamin-Verbindungen der oben genannten Strukturklassen.

Alternativ kann es auch bevorzugt sein, dass die Lochtransportmaterial-Matrix gewählt ist aus Bis-Triarylaminverbindungen oder höherwertigen Triarylaminverbindungen, beispielsweise Tetrakis-Triarylaminverbindungen.

Unter einer Triarylamin-Verbindung wird eine Verbindung verstanden, welche eine oder mehrere Triarylamin-Gruppen aufweist. Unter einer Mono-Triarylaminverbindung wird eine Verbindung verstanden, welche eine einzige Triarylamingruppe aufweist. Eine Triarylamingruppe ist eine Gruppe, in der drei Aryl- oder Heteroarylgruppen an ein gemeinsames Stickstoffatom gebunden sind. Bevorzugt enthält eine Mono-Triarylaminverbindung keine weitere Arylaminogruppe. Besonders bevorzugt enthält eine Mono-Triarylaminverbindung keine weitere Aminogruppe. Analog sind Bis-Triarylaminverbindungen und Tetrakis-Triarylaminverbindungen als Verbindungen definiert, welche zwei bzw. vier Triarylamingruppen enthalten.

Bevorzugt weist die Lochtransportschicht A eine Dicke von 100 bis 300 nm auf, besonders bevorzugt von 130 bis 230 nm.

Bevorzugte Lochtransportmaterialien, die in der Lochtransportschicht A enthalten sind, sind Indenofluorenamin-Derivate (z. B. gemäß
WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Spirobifluoren-Tetrakis-Triarylamine, beispielsweise Spiro-TAD oder Spiro-TTB, Fluoren-Amine (z. B. gemäß den noch nicht offengelegten Anmeldungen
EP 12005369.9, EP 12005370.7 und EP 12005371.5), Spiro-Dibenzopyran-Amine (z. B. gemäß WO 2013/083216) und Dihydroacridin-Derivate (z. B. gemäß WO 2012/150001).

Bevorzugt ist das Lochtransportmaterial gewählt aus Mono-Triarylaminverbindungen, besonders bevorzugt aus Mono-Triarylamin-Verbindungen der oben genannten Strukturklassen.

Alternativ kann es auch bevorzugt sein, dass das Lochtransportmaterial gewählt ist aus Bis-Triarylaminverbindungen oder höherwertigen Triarylaminverbindungen, beispielsweise Tetrakis-Triarylaminverbindungen.

Gemäß einer bevorzugten Ausführungsformenthält die Lochtransportschicht A als Lochtransportmaterial dieselbe Verbindung wie die Lochtransportschicht A' als Lochtransportmaterial-Matrix.

Weiterhin bevorzugt enthält die Lochtransportschicht A bevorzugt keinen p-Dotanden. Besonders bevorzugt enthält sie eine einzige Verbindung, stellt also keine gemischte Schicht dar.

Die Lochtransportschicht B ist erfindungsgemäß p-dotiert.

Bevorzugte Lochtransportmaterial-Matrices der Lochtransportschicht B gehören denselben Strukturklassen an wie oben für Lochtransportschicht A beschrieben. Insbesondere sind dies Indenofluorenamin-Derivate (z. B. gemäß WO 06/122630 oder WO 06/100896), die in
EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Spirobifluoren-Tetrakis-Triarylamine, beispielsweise Spiro-TAD oder Spiro-TTB, Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß
WO 2013/083216) und Dihydroacridin-Derivate (z. B. gemäß
WO 2012/150001).

Bevorzugt ist das Lochtransportmaterial der Schicht B gewählt aus Mono-Triarylaminverbindungen, besonders bevorzugt aus Mono-Triarylamin-Verbindungen der oben genannten Strukturklassen.

Besonders bevorzugte Ausführungsformen von p-Dotanden, insbesondere für die p-dotierten Lochtransportschichten A' und B, sind die in WO 2011/073149, EP 1968131, EP 2276085, EP 2213662, EP 1722602, EP 2045848, DE 102007031220, US 8044390, US 8057712,
WO 2009/003455, WO 2010/094378, WO 2011/120709,
US 2010/0096600 und WO 2012/095143 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden inbesondere die folgenden Verbindungen:

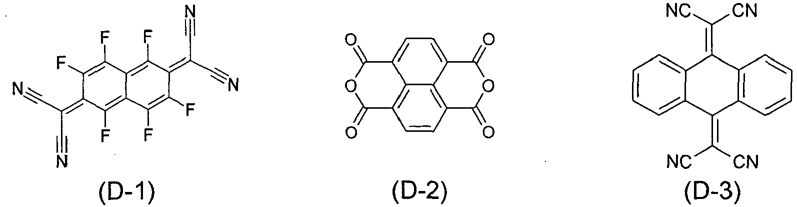

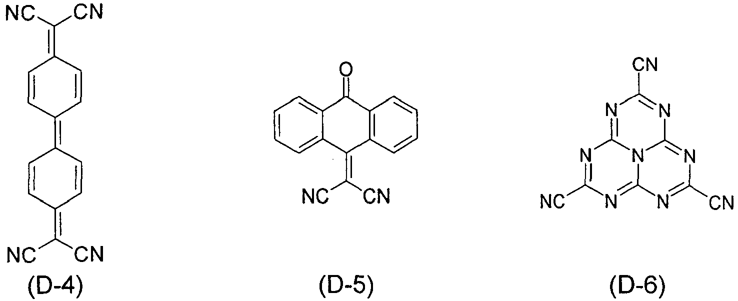

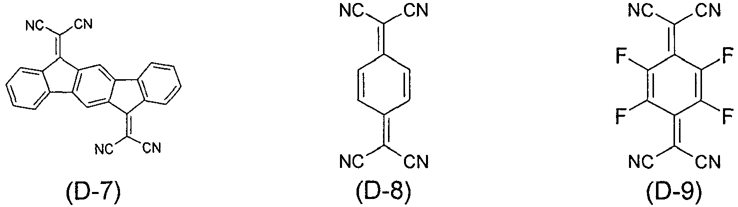

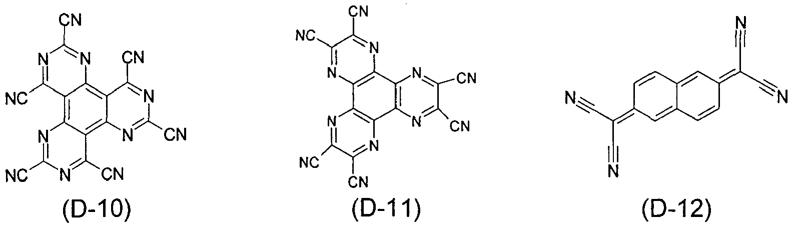

Der p-Dotand in Lochtransportschicht B liegt bevorzugt in einer Konzentration von 0.1 bis 20 Vol-%, bevorzugt 0.5 bis 12 Vol-%, besonders bevorzugt 1 bis 8 Vol-% und ganz besonders bevorzugt 2 bis 6 Vol-% vor.

Die Lochtransportschicht B weist bevorzugt eine Dicke von 5 bis 50 nm, besonders bevorzugt 10 bis 40 nm, auf.

Die Lochtransportschicht C enthält bevorzugt keinen p-Dotanden. Besonders bevorzugt enthält sie eine einzige Verbindung, stellt also keine gemischte Schicht dar.

Gemäß einer bevorzugten Ausführungsform schließt sich die Lochtransportschicht C in unmittelbarem Kontakt anodenseitig an die emittierende Schicht an.

Bevorzugte Lochtransportmaterialien der Lochtransportschicht C gehören denselben Strukturklassen an wie oben für Lochtransportschicht A beschrieben. Insbesondere sind dies Indenofluorenamin-Derivate (z. B. gemäß WO 06/122630 oder WO 06/100896), die in
EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Spirobifluoren-Tetrakis-Triarylamine, beispielsweise Spiro-TAD oder Spiro-TTB, Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß
WO 2013/083216) und Dihydroacridin-Derivate (z. B. gemäß
WO 2012/150001).

Bevorzugt ist das Lochtransportmaterial der Schicht C gewählt aus Mono-Triarylaminverbindungen,
besonders bevorzugt aus Mono-Triarylamin-Verbindungen der oben genannten Strukturklassen.

Die Lochtransportschicht C weist bevorzugt eine Dicke von 5 bis 50 nm, besonders bevorzugt 10 bis 40 nm, auf.

Gemäß einer bevorzugten Ausführungsform sind die Lochtransportmaterialien der Lochtransportschichten A und C unterschiedlich.

Es ist bevorzugt, dass das HOMO des Lochtransportmaterials der Lochtransportschicht C zwischen -4,9 und -5,6 eV, bevorzugt zwischen -5,0 und -5,5 eV, und besonders bevorzugt zwischen -5,1 bis -5,4 eV liegt.

Es ist bevorzugt, dass das HOMO des Lochtransportmaterials der Lochtransportschicht A um einen Betrag von mindestens 0,2 eV, bevorzugt mindestens 0,3 eV, besonders bevorzugt mindestens 0,4 eV, höher liegt als das HOMO des Lochtransportmaterials der Lochtransportschicht C.

Bevorzugt liegt der Wert für das HOMO des Lochtransportmaterials der Lochtransportschicht A zwischen -4.7 und -5.4 eV, bevorzugt zwischen -4.8 und -5.3 eV, und besonders bevorzugt zwischen -4.9 eV und -5.2 eV.

Dabei wird das HOMO (highest occupied molecular orbital) durch quantenchemische Rechnungen bestimmt und anhand von CyclovoltammetrieMessungen kalibriert, wie bei den Ausführungsbeispielen näher erläutert wird.

Gemäß einer weiteren bevorzugten Ausführungsform weist die Lochtransportschicht B als Lochtransportmaterial-Matrix dieselbe Verbindung auf wie die Lochtransportschicht C als Lochtransportmaterial.

In einer weiteren bevorzugten Ausführungsform ist in der Lochtransportschicht A eine Bis-Triarylamin-Verbindung oder höherwertige Triarylamin-Verbindung enthalten, beispielsweise eine Tetrakis-Triarylaminverbindung, und in der Lochtransportschicht C ist eine Mono-Triarylamin-Verbindung enthalten. Besonders bevorzugt ist in der Lochtransportschicht A eine Bis-Triarylamin-Verbindung oder höherwertige Triarylamin-Verbindung enthalten, beispielsweise eine Tetrakis-Triarylaminverbindung, und in den Lochtransportschichten B und C ist eine Mono-Triarylamin-Verbindung enthalten.

Es ist erfindungsgemäß bevorzugt, dass die emittierende Schicht oder eine der emittierenden Schichten unmittelbar an die Lochtransportschicht C angrenzt.

Bevorzugt enthalten sie jeweils eine oder mehrere gleiche oder verschiedene Mono-Triarylamin-Verbindungen.

Es ist weiterhin bevorzugt, dass mindestens eine der Lochtransportschichten A, B, C und A' mindestens eine Verbindung gemäß einer der Formeln 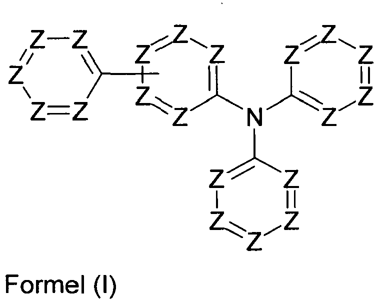 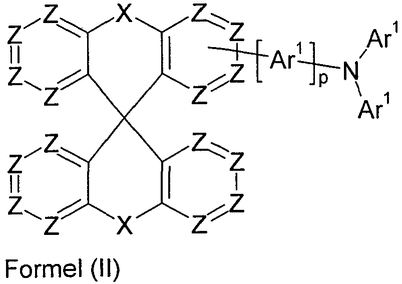 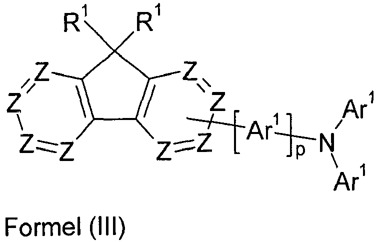 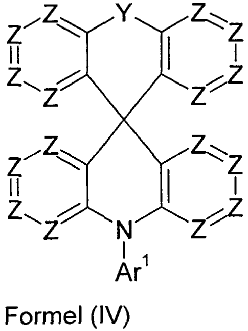 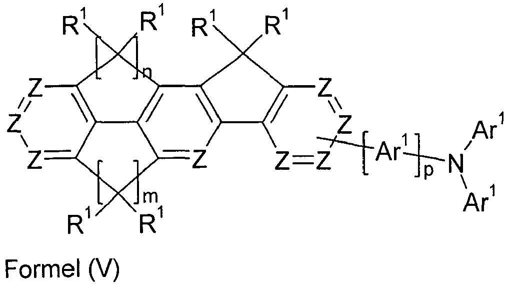 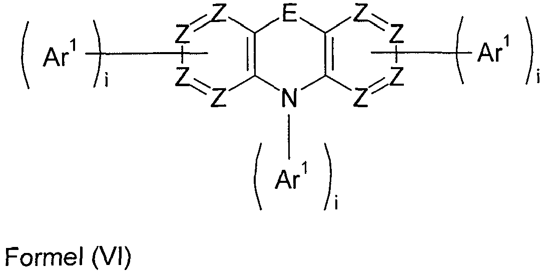 enthält, wobei gilt:
- Z: ist bei jedem Auftreten gleich oder verschieden N oder CR¹, wobei Z gleich C ist, wenn ein Substituent gebunden ist;
- X,Y: sind bei jedem Auftreten gleich oder verschieden eine Einfachbindung, O, S, Se, BR¹, C(R¹)₂, Si(R¹)₂, NR¹, PR¹, C(R¹)₂-C(R¹)₂, oder CR¹=CR¹;
- E ist: O, S, Se, BR¹, C(R¹)₂, Si(R¹)₂, NR¹, PR¹, C(R¹)₂-C(R¹)₂, oder CR¹=CR¹;
- Ar¹: ist bei jedem Auftreten gleich oder verschieden ein aromatisches oder heteroaromatisches Ringsystem mit 5 bis 60 aromatischen Ringatomen, welches durch einen oder mehrere Reste R¹ substituiert sein kann; und
- R¹: ist bei jedem Auftreten gleich oder verschieden H, D, F, Cl, Br, I, CHO, C(=O)R², P(=O)(R²)₂, S(=O)R², S(=O)₂R², CR²=CR²R², CN, NO₂, Si(R²)₃, OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C , Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ring-atomen, das jeweils durch einen oder mehrere Reste R² substituiert sein kann, oder eine Kombination dieser Systeme; dabei können zwei oder mehrere benachbarte Substituenten R¹ auch miteinander ein mono- oder polycyclisches, aliphatisches oder aromatisches Ringsystem bilden;
- R²: ist bei jedem Auftreten gleich oder verschieden H, D, CN oder ein aliphatischer, aromatischer und/oder heteroaromatischer Kohlenwasserstoffrest mit 1 bis 20 C-Atomen, in dem auch H-Atome durch D oder F ersetzt sein können; dabei können zwei oder mehrere benachbarte Substituenten R² auch miteinander ein mono- oder polycyclisches, aliphatisches oder aromatisches Ringsystem bilden;
- i: bei jedem Auftreten gleich oder verschieden 0 oder 1 ist, wobei die Summe aller i mindestens gleich 1 ist;
- p: gleich 0 oder 1 ist;
- m, n: gleich oder verschieden 0 oder 1 sind, wobei die Summe aus m und n gleich 1 oder 2 ist.

Bevorzugt enthalten mindestens zwei der Lochtransportschichten A, B, C und A' mindestens eine Verbindung gemäß einer der Formeln (I) bis (VI), besonders bevorzugt mindestens drei der Lochtransportschichten A, B, C und A', und ganz besonders bevorzugt alle der Lochtransportschichten A, B, C und A'.

In Lochtransportschicht A werden bevorzugt Verbindungen der Formeln (I), (II), (III) und (V) eingesetzt.

Für die oben genannten Formeln (I) bis (VI) gilt bevorzugt, dass nicht mehr als drei Gruppen Z in einem Ring gleich N sind. Es gilt allgemein als bevorzugt, dass Z gleich CR¹ ist.

Die Gruppe X ist bei jedem Auftreten bevorzugt gleich oder verschieden gewählt aus einer Einfachbindung, C(R¹)₂, O und S, besonders bevorzugt ist sie eine Einfachbindung.

Die Gruppe Y ist bevorzugt gewählt aus O und C(R¹)₂, besonders bevorzugt ist sie O.

Die Gruppe E ist bevorzugt gewählt aus C(R¹)₂, O und S, besonders bevorzugt ist sie C(R¹)₂.

Die Gruppe Ar¹ ist bei jedem Auftreten gleich oder verschieden gewählt aus aromatischen oder heteroaromatischen Ringsystemen mit 6 bis 30 aromatischen Ringatomen, die mit einem oder mehreren Resten R¹ substituiert sein können. Besonders bevorzugt ist Ar¹ gewählt aus Aryl- oder Heteroarylgruppen mit 6 bis 18 aromatischen Ringatomen, die mit einem oder mehreren Resten R¹ substituiert sein können.

R¹ ist bei jedem Auftreten gleich oder verschieden gewählt aus H, D, F, Cl, Br, I, C(=O)R², CN, Si(R²)₃, N(R²)₂, NO₂, P(=O)(R²)₂, S(=O)R², S(=O)₂R², einer geradkettige Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R² substituiert sein können und wobei eine oder mehrere CH₂-Gruppen in den oben genannten Gruppen durch -R²C=CR²-, -C≡C-, Si(R²)₂, C=O, C=S, C=NR², -C(=O)O-, -C(=O)NR²-, NR², P(=O)(R²), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 5 bis 30 aromatischen Ringatomen, das jeweils durch einen oder mehrere Reste R² substituiert sein kann, wobei zwei oder mehr Reste R¹ miteinander verknüpft sein können und einen Ring bilden können.

### Folgende Definitionen gelten allgemein:

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Beispiele für bevorzugte Lochtransportmaterialien zur Verwendung in der elektronischen Vorrichtung gemäß der vorliegenden Erfindung, insbesondere in den Schichten A', A, B und C, sind im Folgenden gezeigt.

| | | |
|---|---|---|
| 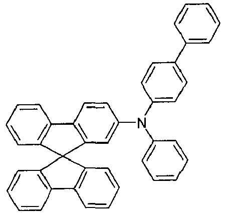 | 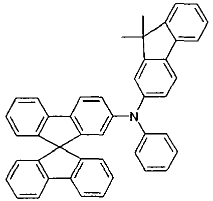 | 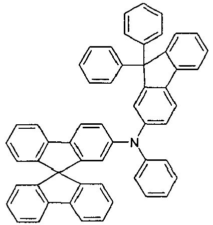 |
| (1) | (2) | (3) |
| 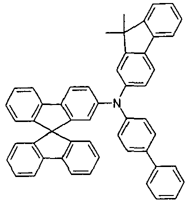 | 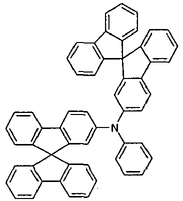 | 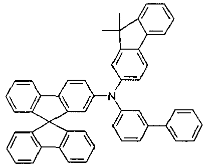 |
| (4) | (5) | (6) |
| 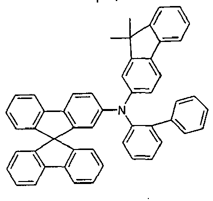 | 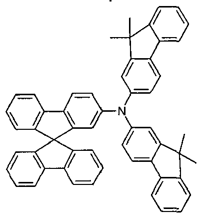 | 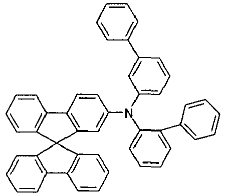 |
| (7) | (8) | (9) |
| 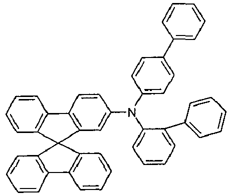 | 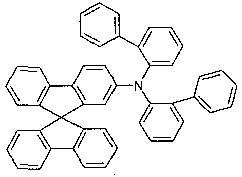 | 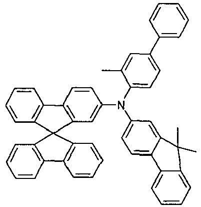 |
| (10) | (11) | (12) |
| 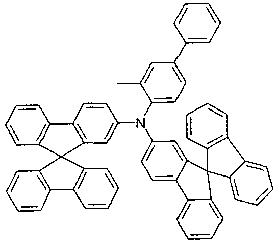 | 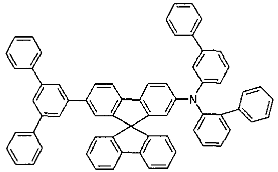 | 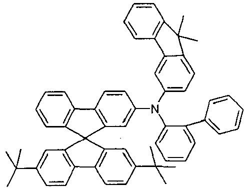 |
| (13) | (14) | (15) |
| 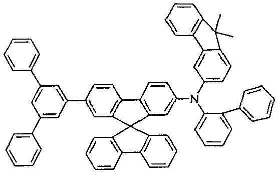 | 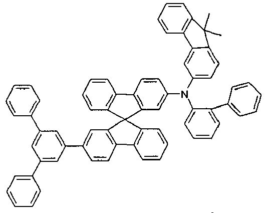 | 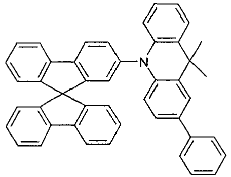 |
| (16) | (17) | (18) |
| 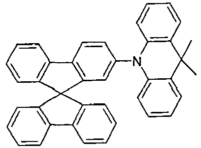 | 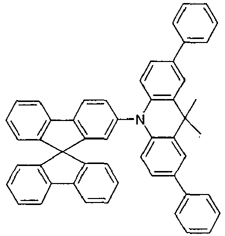 | 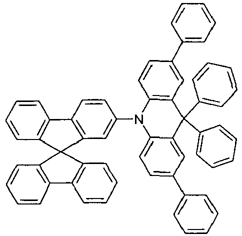 |
| (19) | (20) | (21) |
| 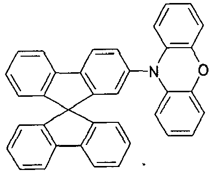 | 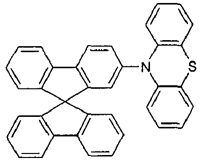 | 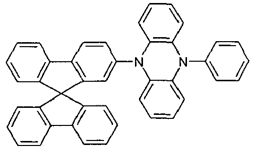 |
| (22) | (23) | (24) |
| 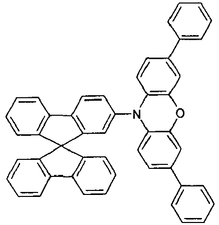 | 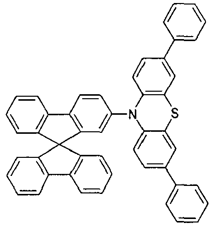 | 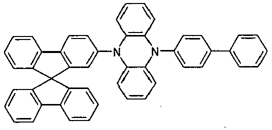 |
| (25) | (26) | (27) |
| 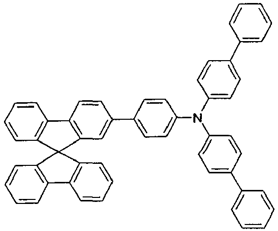 | 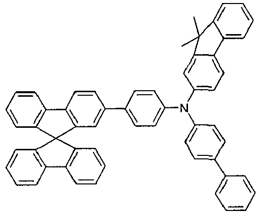 | 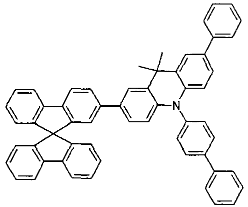 |
| (28) | (29) | (30) |
| 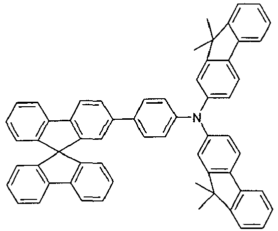 | 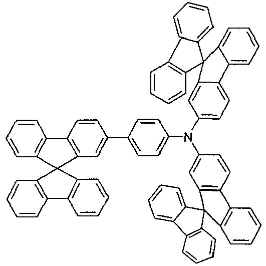 | 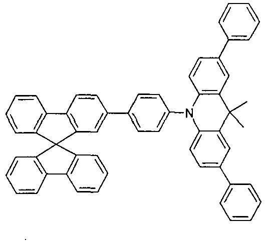 |
| (31) | (32) | (33) |
| 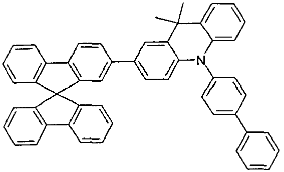 | 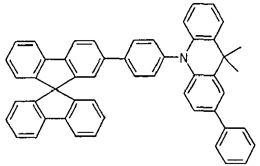 | 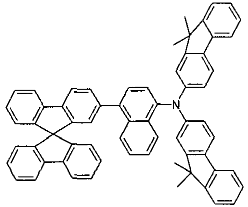 |
| (34) | (35) | (36) |
| 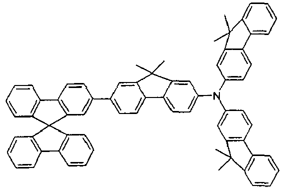 | 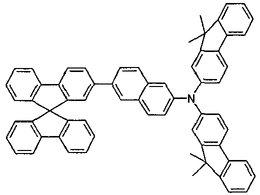 | 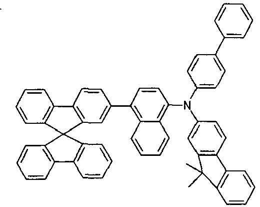 |
| (37) | (38) | (39) |
| 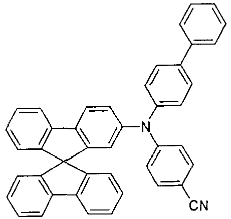 | 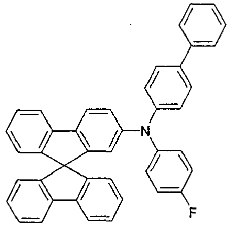 | 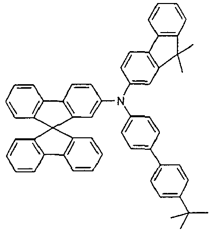 |
| (40) | (41) | (42) |
| 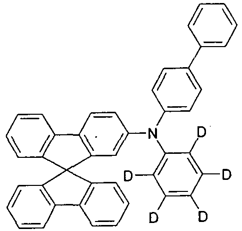 | 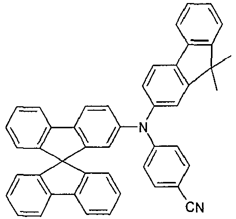 | 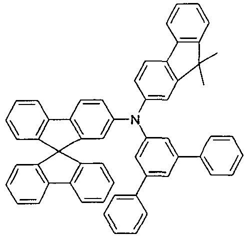 |
| (43) | (44) | (45) |
| 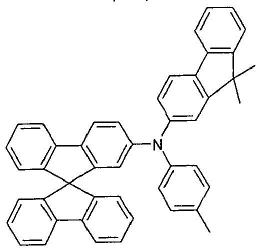 | 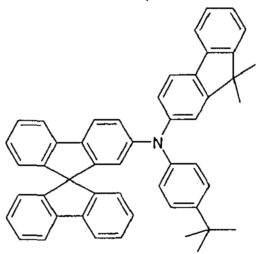 | 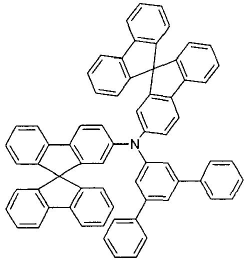 |
| (46) | (47) | (48) |
| 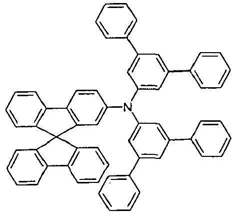 | 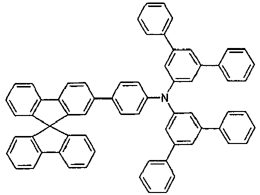 | 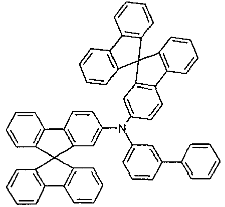 |
| (49) | (50) | (51) |
| 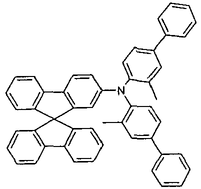 | 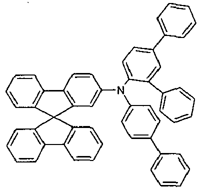 | 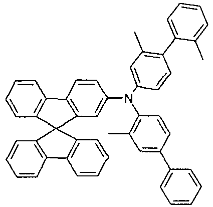 |
| (52) | (53) | (54) |
| 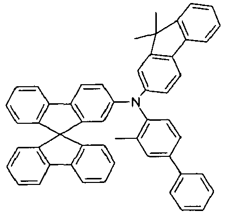 | 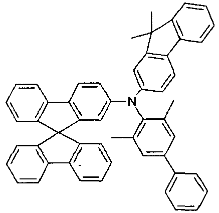 | 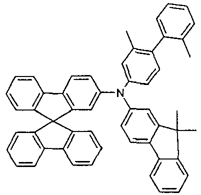 |
| (55) | (56) | (57) |
| 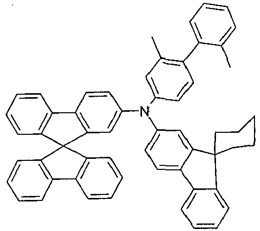 | 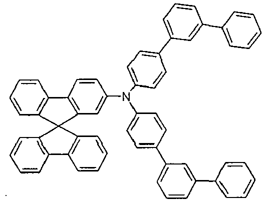 | 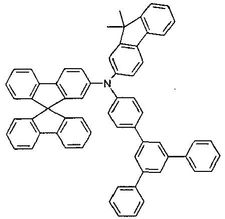 |
| (58) | (59) | (60) |
| 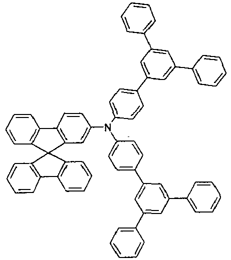 | 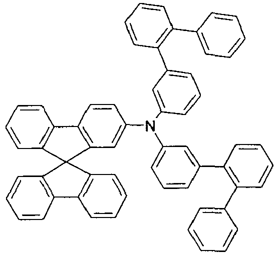 | 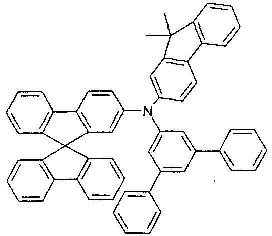 |
| (61) | (62) | (63) |
| 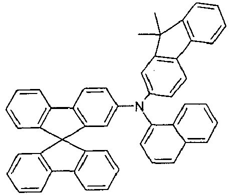 | 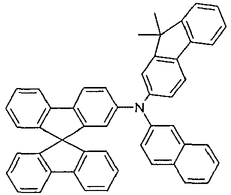 | 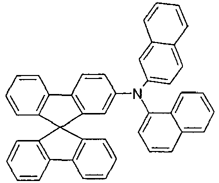 |
| (64) | (65) | (66) |
| 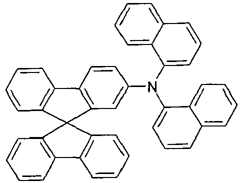 | 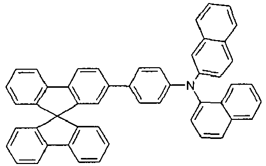 | 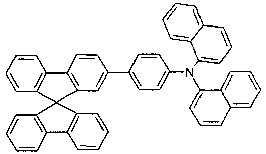 |
| (67) | (68) | (69) |
| 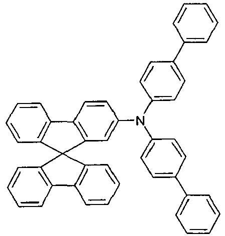 | 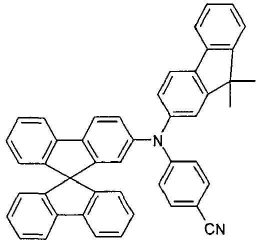 | 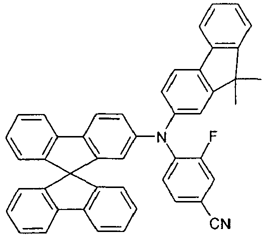 |
| (70) | (71) | (72) |
| 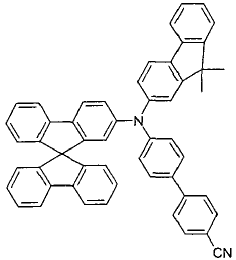 | 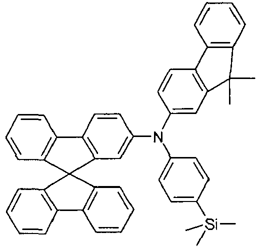 | 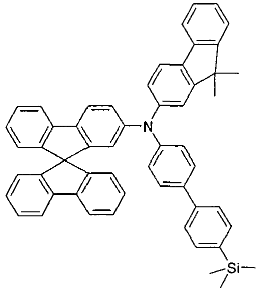 |
| (73) | (74) | (75) |
| 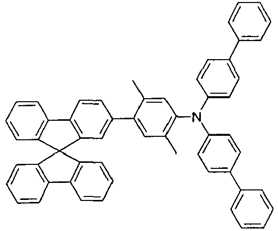 | 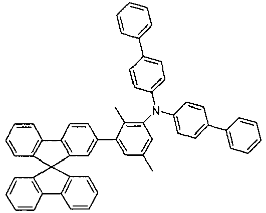 | 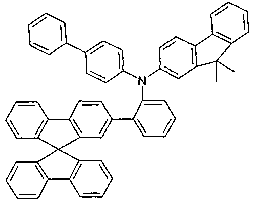 |
| (76) | (77) | (78) |
| 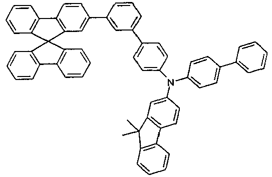 | 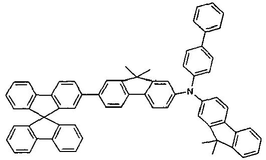 | 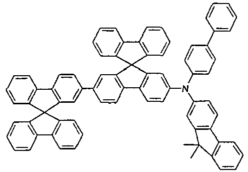 |
| (79) | (80) | (81) |
| 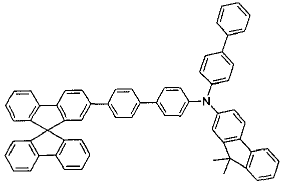 | 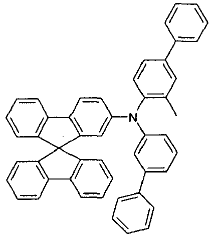 | 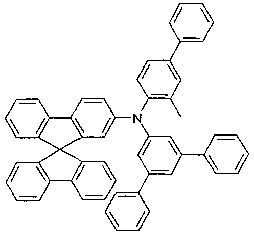 |
| (82) | (83) | (84) |
| 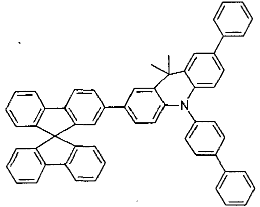 | 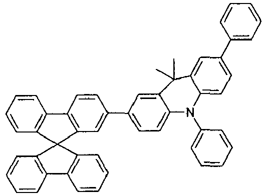 | 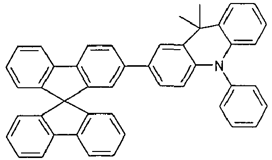 |
| (85) | (86) | (87) |
| 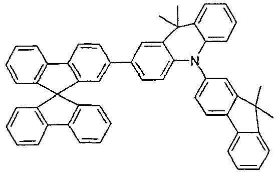 | 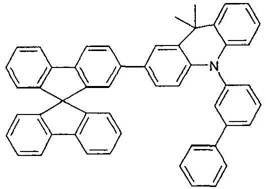 | 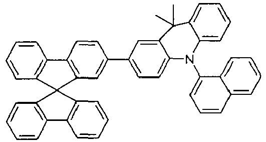 |
| (88) | (89) | (90) |
| 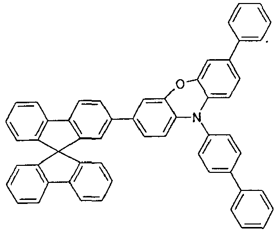 | 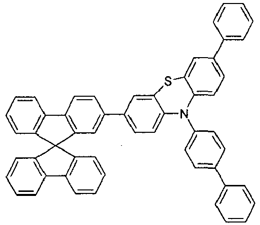 | 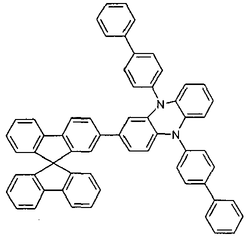 |
| (91) | (92) | (93) |
| 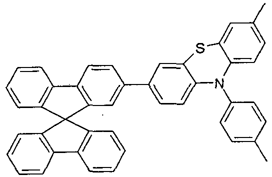 | 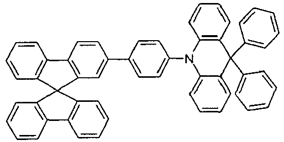 | 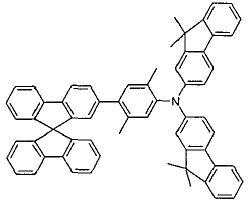 |
| (94) | (95) | (96) |
| 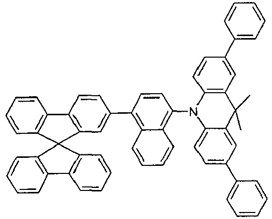 | 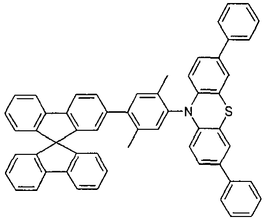 | 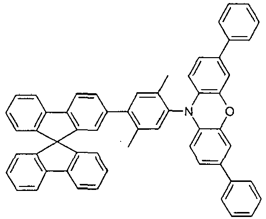 |
| (97) | (98) | (99) |
| 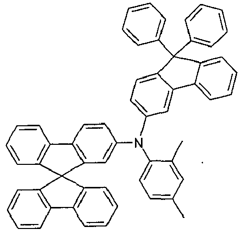 | 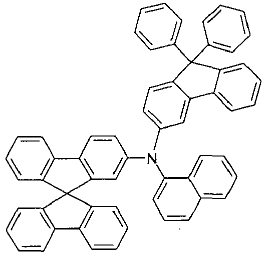 | 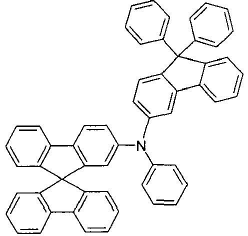 |
| (100) | (101) | (102) |
| 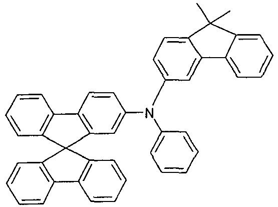 | 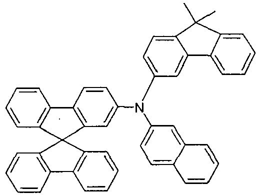 | 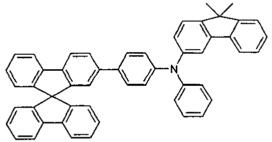 |
| (103) | (104) | (105) |
| 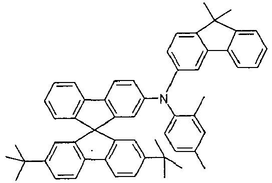 | 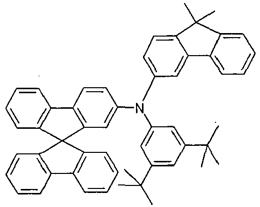 | 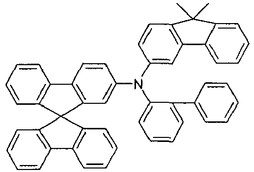 |
| (106) | (107) | (108) |
| 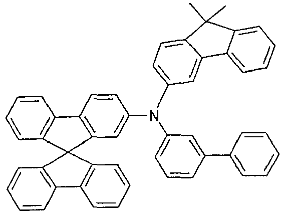 | 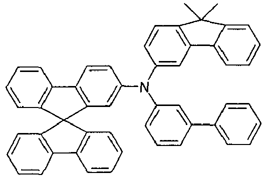 | 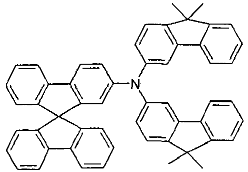 |
| (109) | (110) | (111) |
| 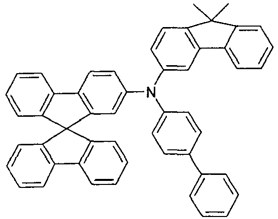 | 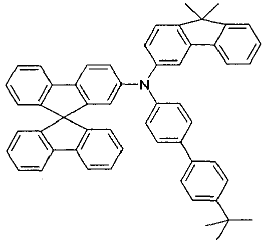 | 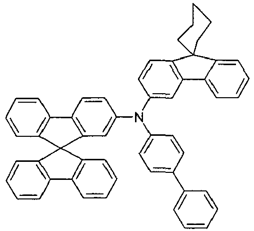 |
| (112) | (113) | (114) |
| 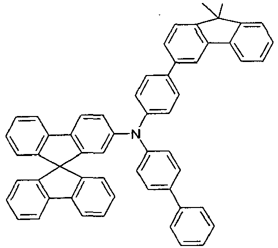 | 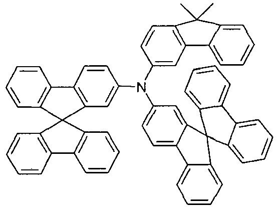 | 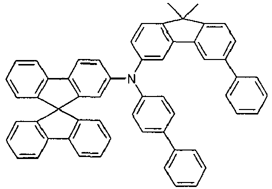 |
| (115) | (116) | (117) |
| 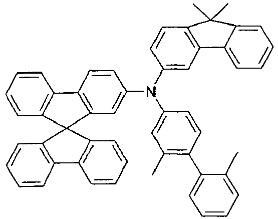 | 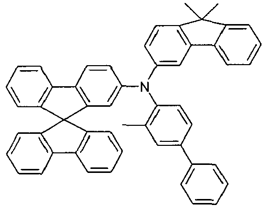 | 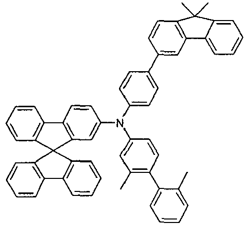 |
| (118) | (119) | (120) |
| 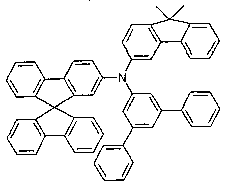 | 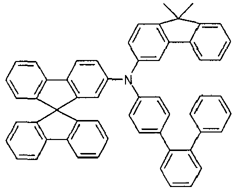 | 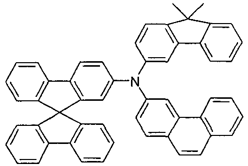 |
| (121) | (122) | (123) |
| 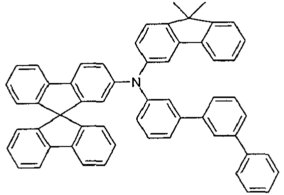 | 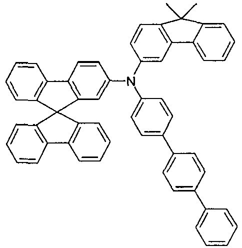 | 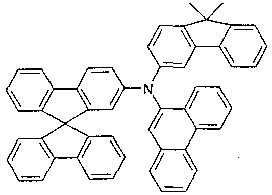 |
| (124) | (125) | (126) |
| 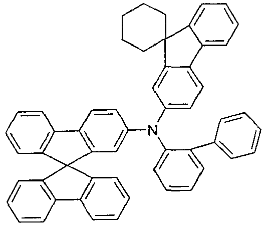 | 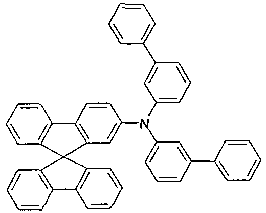 | 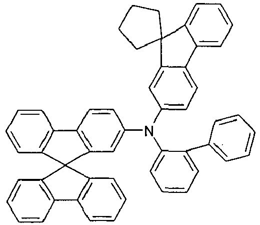 |
| (127) | (128) | (129) |
| 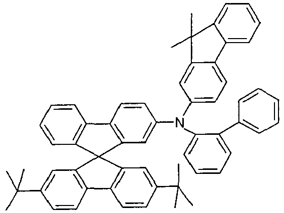 | 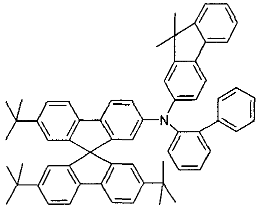 | 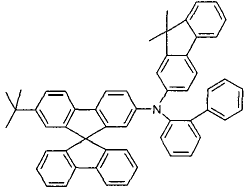 |
| (130) | (131) | (132) |
| 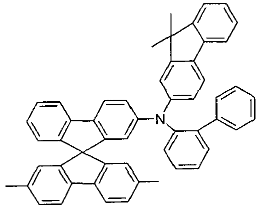 | 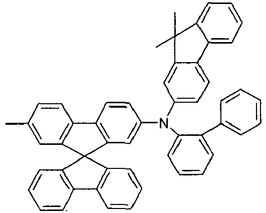 | 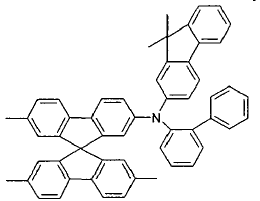 |
| (133) | (134) | (135) |
| 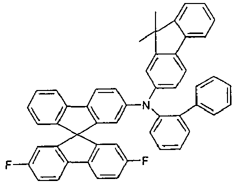 | 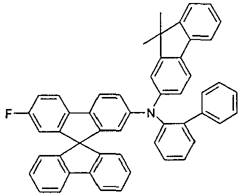 | 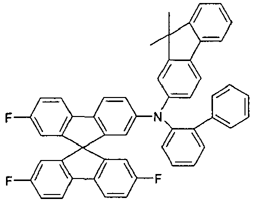 |
| (136) | (137) | (138) |
| 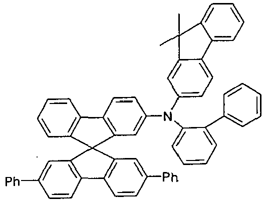 | 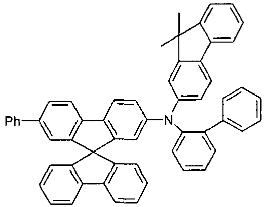 | 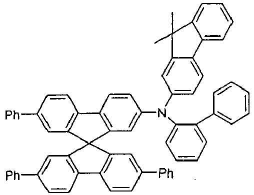 |
| (139) | (140) | (141) |
| 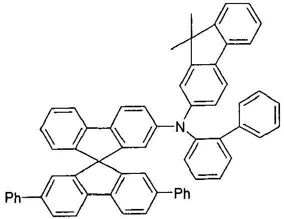 | 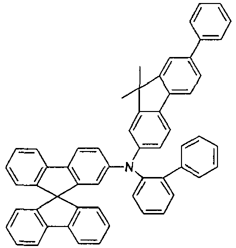 | 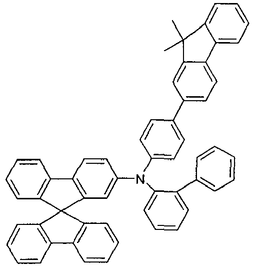 |
| (142) | (143) | (144) |
| 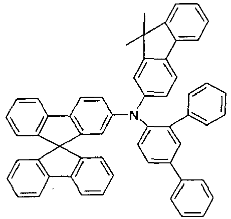 | 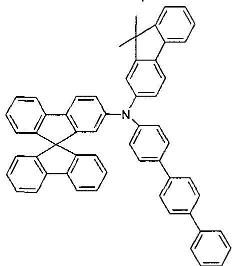 | 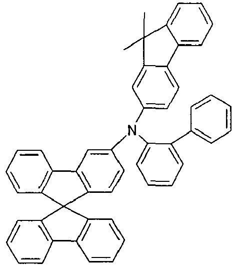 |
| (145) | (146) | (147) |
| 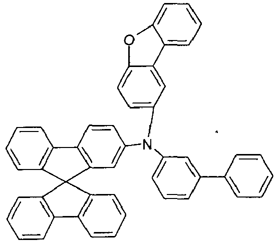 | 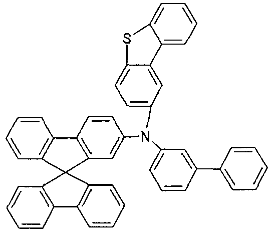 | 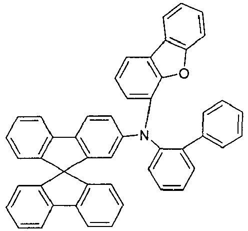 |
| (148) | (149) | (150) |
| 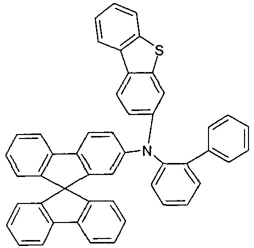 | 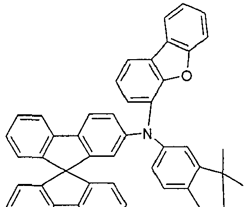 | 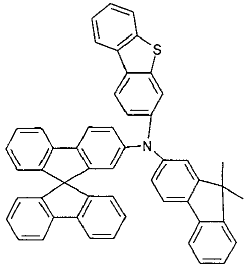 |
| (151) | (152) | (153) |
| 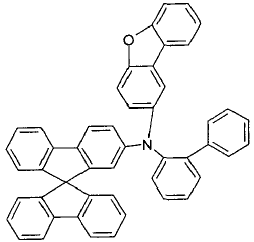 | 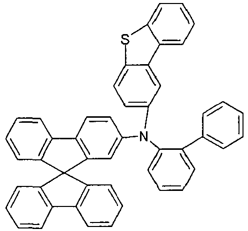 | 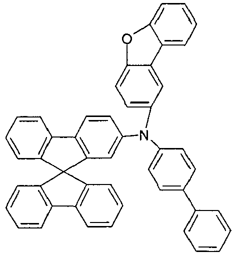 |
| (154) | (155) | (156) |
| 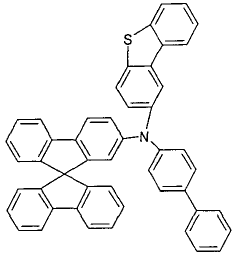 | 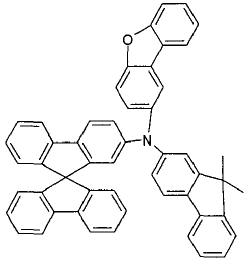 | 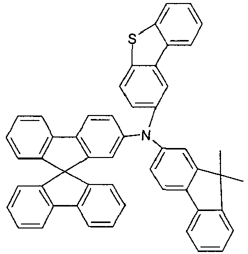 |
| (157) | (158) | (159) |
| 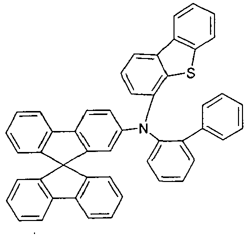 | 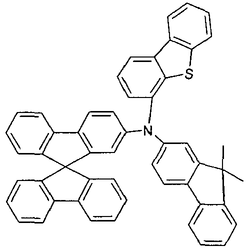 | 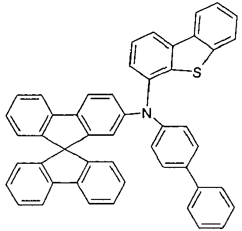 |
| (160) | (161) | (162) |
| 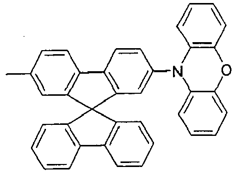 | 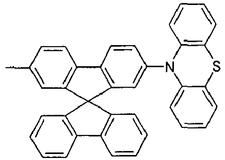 | 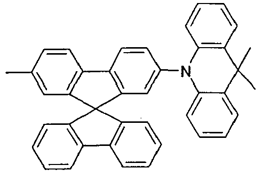 |
| (163) | (164) | (165) |
| 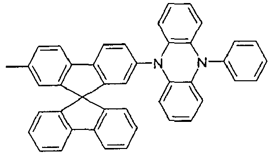 | 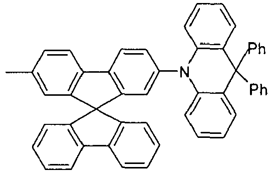 | 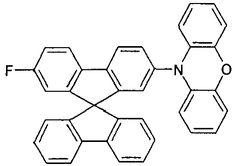 |
| (166) | (167) | (168) |
| 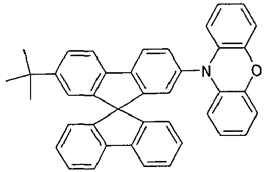 | 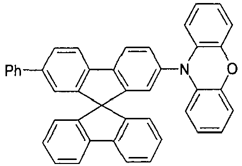 | 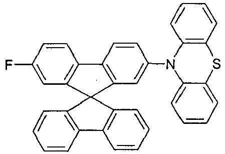 |
| (169) | (170) | (171) |
| 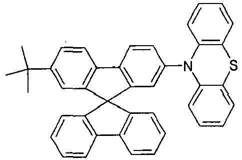 | 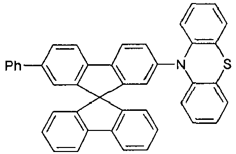 | 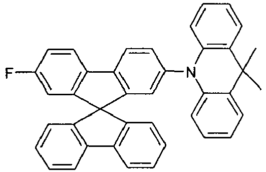 |
| (172) | (173) | (174) |
| 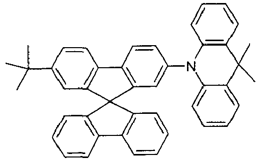 | 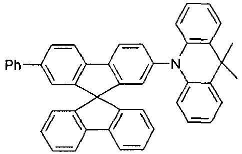 | 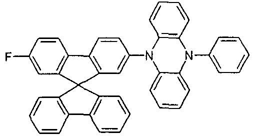 |
| (175) | (176) | (177) |
| 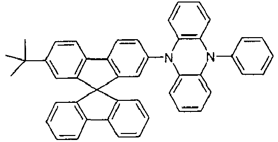 | 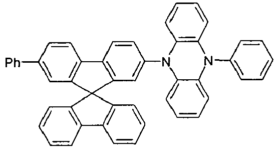 | 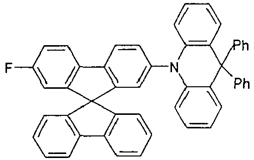 |
| (178) | (179) | (180) |
| 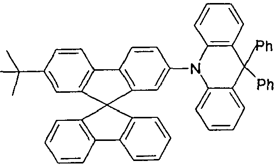 | 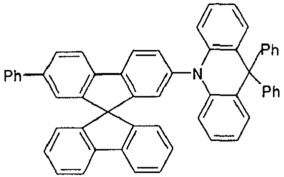 | 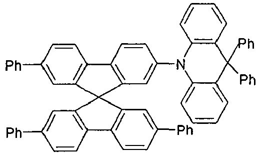 |
| (181) | (182) | (183) |
| 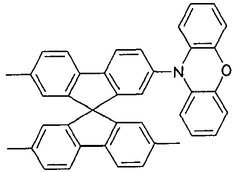 | 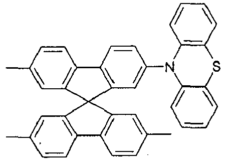 | 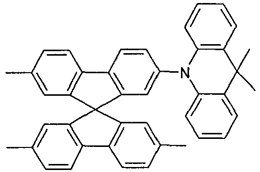 |
| (184) | (185) | (186) |
| 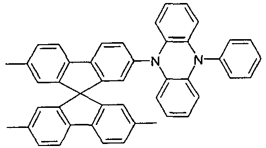 | 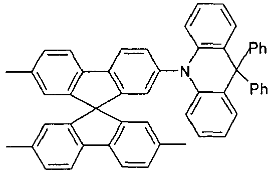 | 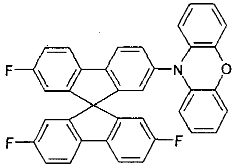 |
| (187) | (188) | (189) |
| 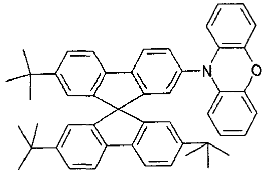 | 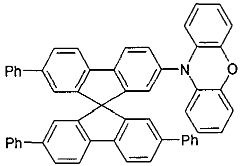 | 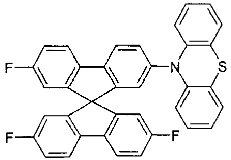 |
| (190) | (191) | (192) |
| 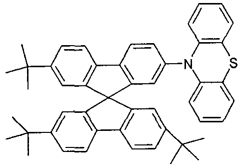 | 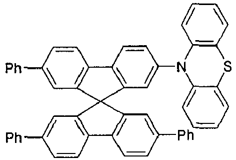 | 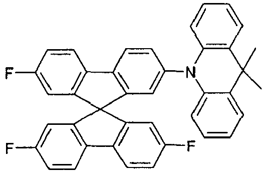 |
| (193) | (194) | (195) |
| 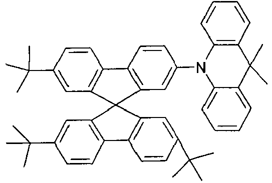 | 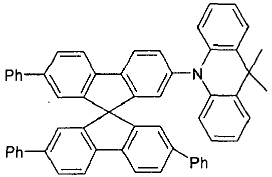 | 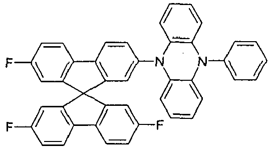 |
| (196) | (197) | (198) |
| 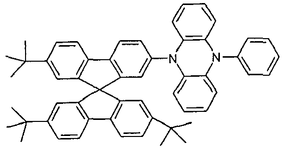 | 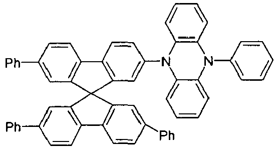 | 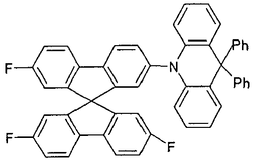 |
| (199) | (200) | (201) |
| 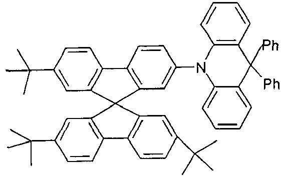 | 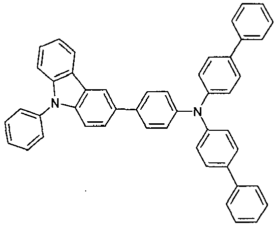 | |
| (202) | (203) | |

Die erfindungsgemäße elektronische Vorrichtung kann eine oder mehrere emittierende Schichten enthalten. Die emittierenden Schichten können fluoreszierend oder phosphoreszierend sein, das heißt fluoreszierende oder phosphoreszierende Emitter enthalten.

Vom Begriff phosphoreszierende Emitter (Dotanden) sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Emitter eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen phosphoreszierenden Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

Bevorzugte fluoreszierende Emitter zur Verwendung in den erfindungsgemäßen elektronischen Vorrichtungen sind gewählt aus der Klasse der Triarylamin-Verbindungen, wie oben definiert. Bevorzugt ist mindestens eine der Aryl- oder Heteroarylgruppen, die an das Stickstoffatom gebunden sind, ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte Dotanden sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß
WO 2007/140847, sowie die in WO 2010/012328 offenbarten Indenofluorenderivate mit kondensierten Arylgruppen. Ebenfalls bevorzugt sind die in WO 2012/048780 und der noch nicht offengelegten EP 12004426.8 offenbarten Pyren-Arylamine. Ebenfalls bevorzugt sind die in der noch nicht offengelegten EP 12006239.3 offenbarten Benzoindenofluoren-Amine und die in der noch nicht offengelegten EP 13000012.8 offenbarten Benzofluoren-Amine.

Die emittierende Schicht enthält bevorzugt ein oder mehrere Hostmaterialien (Matrixmaterialien) sowie ein oder mehrere Dotandmaterialien (Emittermaterialien).

Eine emittierende Schicht enthält gemäß einer bevorzugten Ausführungsform mehrere Matrixmaterialien (Mixed-Matrix-Systeme) und/oder mehrere Dotanden. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In Mixed-Matrix-Systemen stellt bevorzugt eines der beiden Matrixmaterialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Bevorzugte Ausführungsformen von Mixed-Matrix-Systemen sind unter anderem in der Anmeldung WO 2010/108579 offenbart.

Die Mixed-Matrix-Systeme können einen oder mehrere Dotanden umfassen, bevorzugt einen oder mehrere phosphoreszierende Dotanden. Allgemein werden Mixed-Matrix-Systeme bevorzugt in phosphoreszierenden emittierenden Schichten eingesetzt.

Bevorzugte Matrixmaterialien für fluoreszierende Emitter sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Emitter sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß
WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Die erfindungsgemäße elektronische Vorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei bevorzugt mindestens eine dieser Schichten mindestens eine Verbindung gemäß Formel (I) enthält und wobei die drei Schichten blaue, grüne und orange oder rote Emission zeigen (für den prinzipiellen Aufbau siehe z. B.
WO 2005/011013). Alternativ und/oder zusätzlich können die erfindungsgemäßen Verbindungen auch in der Lochtransportschicht oder in einer anderen Schicht vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Als Kathode der erfindungsgemäßen elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Die erfindungsgemäße elektronische Vorrichtung enthält neben Anode, Kathode, emittierender Schicht und den Lochtransportschichten A, B, C und wahlweise Lochtransportschicht A' bevorzugt noch weitere Funktionsschichten.

Die Abfolge der Schichten der elektronischen Vorrichtung ist bevorzugt die folgende:
Anode-Lochtransportschicht A'-Lochtransportschicht A-Lochtransportschicht B-Lochtransportschicht C-emittierende Schicht-Elektronentransportschicht-Elektroneninjektionsschicht-Kathode.
Es müssen nicht alle der genannten Schichten vorhanden sein, und/oder es können zusätzlich zu den genannten Schichten weitere Schichten vorhanden sein.

Diese zusätzlichen Schichten sind bevorzugt gewählt aus Lochinjektionsschichten, Lochtransportschichten, Elektronenblockierschichten, emittierenden Schichten, Zwischenschichten (Interlayers), Elektronentransportschichten, Elektroneninjektionsschichten, Lochblockierschichten, Excitonenblockierschichten, Ladungserzeugungsschichten (charge generating layers), p/n-Übergängen und Auskopplungsschichten.

Die erfindungsgemäße elektronische Vorrichtung weist bevorzugt mindestens eine Elektronentransportschicht auf, welche zwischen emittierender Schicht und Kathode angeordnet ist, wobei die Elektronentransportschicht bevorzugt mindestens einen n-Dotanden und mindestens eine Elektronentransportmaterial-Matrix enthält.

Unter einem n-Dotanden wird eine Verbindung verstanden, welche die andere in der Schicht vorhandene Verbindung (die Matrix) zumindest teilweise reduzieren kann und auf diese Weise die Leitfähigkeit der Schicht erhöht. Typischerweise sind n-Dotanden gemäß der vorliegenden Anmeldung Elektronendonatorverbindungen bzw. starke Reduktionsmittel. Als n-Dotanden können beispielsweise die in Chem. Rev. 2007, 107, S. 1233 ff., Abschnitt 2.2, offenbarten Materialien verwendet werden, wie Alkalimetalle, Erdalkalimetalle und elektronenreiche und leicht oxidierbare organische Verbindungen oder Übergangsmetallkomplexe.

Weiterhin weist die erfindungsgemäße elektronische Vorrichtung bevorzugt mindestens eine Elektroneninjektionsschicht aus, welche zwischen Elektronentransportschicht und Kathode angeordnet ist. Bevorzugt grenzt die Elektroneninjektionsschicht unmittelbar an die Kathode an.

Als Materialien für die Elektronentransportschicht und Elektroneninjektionsschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Während der Herstellung wird die Vorrichtung bevorzugt strukturiert, kontaktiert und schließlich versiegelt, um Wasser und/oder Luft auszuschließen.

In einer bevorzugten Ausführungsform ist die erfindungsgemäße elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls, dass in der erfindungsgemäßen elektronischen Vorrichtung eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Bevorzugt ist ebenfalls, dass in der erfindungsgemäßen elektronischen Vorrichtung eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden.

Weiterhin bevorzugt ist es, dass zur Herstellung der erfindungsgemäßen elektronischen Vorrichtung eine oder mehrere Schichten aus Lösung und eine oder mehrere Schichten durch ein Sublimationsverfahren aufgetragen werden.

Die erfindungsgemäßen elektronischen Vorrichtungen können in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### Teil A: Bestimmung der HOMO-Lagen von Verbindungen

Die HOMO-Lagen der Materialien werden über quantenchemische Rechnungen bestimmt. Hierzu wird das Programmpaket "Gaussian03W" (Gaussian Inc.) verwendet. Zur Berechnung organischer Substanzen ohne Metalle wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SFC/DFT/Default Spin/B3PW91" mit dem Basissatz "6-31G(d)" verwendet (Charge 0, Spin Singlet). Aus der Energierechnung erhält man das HOMO HEh in Hartree-Einheiten. Daraus werden die anhand von Cyclovoltammetriemessungen kalibrierten HOMO-Werte in Elektronenvolt wie folgt bestimmt:
HOMO(eV) = ((HEh*27.212)-0.9899)/1.1206

Diese Werte sind im Sinne dieser Anmeldung als HOMO der Materialien anzusehen.

**Tabelle mit HOMO-Daten der verwendeten Verbindungen (Strukturen s. u.)**

| Material | HOMO |
|---|---|
| HIM1/HTM1 | -5,25 eV |
| HIM 2 | -4,85 eV |
| NPB | -5,16 eV |
| HTM2 | -5,43 eV |
| HTM3 | -5,23 eV |
| HTM4 | -5,35 eV |
| HTM5 | -5,32 eV |
| HTM6 | -5,23 eV |

### Teil B: Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden erfinderischen Beispielen E1 bis E13 und in den Referenzbeispielen V1-V11 werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / p-dotierte Lochtransportschicht A' (HIL1) / Lochtransportschicht A (HTL) / p-dotierte Lochtransportschicht B (HIL2) / Lochtransportschicht C (EBL) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Die zur Herstellung der OLEDs benötigten Materialien sind in Tabelle 1 gezeigt, der Aufbau der verschiedenen hergestellten elektronischen Vorrichtungen in Tabelle 2.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB1(5%) bedeutet hierbei, dass das Material H1 in einem Volumenanteil von 95% und SEB1 in einem Anteil von 5% in der Schicht vorliegt. Analog kann auch die Elektronentransportschicht oder die Lochinjektionsschichten aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 10 mA/cm² bezeichnet die externe Quanteneffizienz bei einer Stromdichte von 10mA/cm². LD80 @ 60 mA/cm² ist die Lebensdauer, bis das OLED bei einer Starthelligkeit bei konstantem Strom von 60mA/cm² auf 80 % der Anfangsintensität abgefallen ist.

| Tabelle 1: Strukturen der verwendeten Materialien | | |
|---|---|---|
| 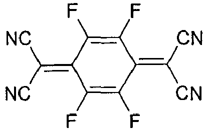 | 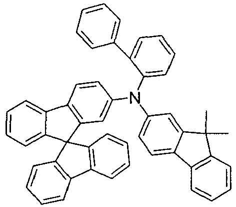 | 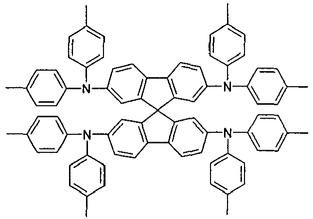 |
| F4TCNQ | HIM1 | HIM2 |
| 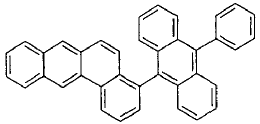 | 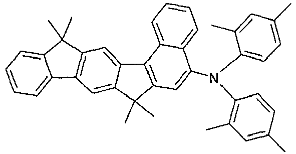 | 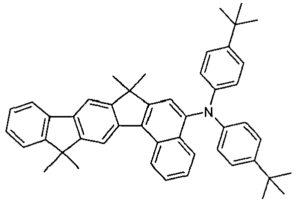 |
| H1 | SEB1 | SEB2 |
| 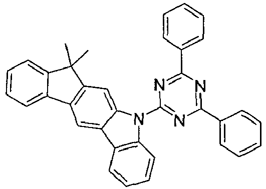 | 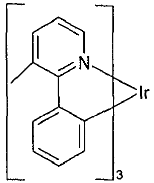 | 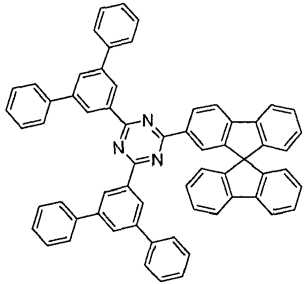 |
| H2 | TEG | ETM |
| 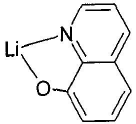 | 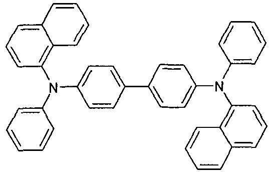 | 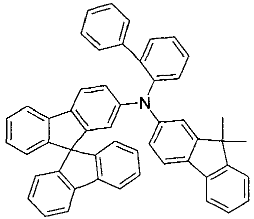 |
| LiQ | NPB | HTM1 |
| 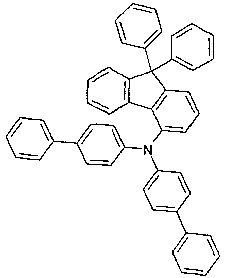 | 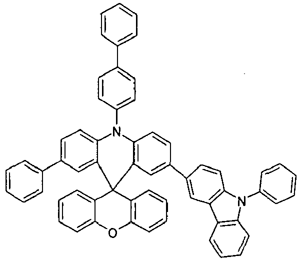 | 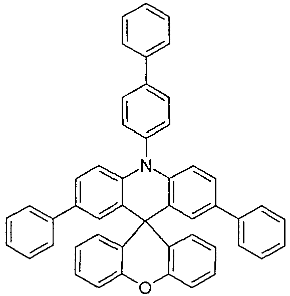 |
| HTM2 | HTM3 | HTM4 |
| 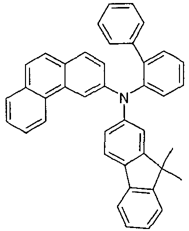 | 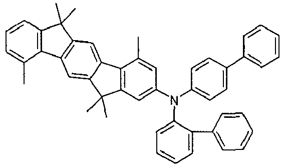 | 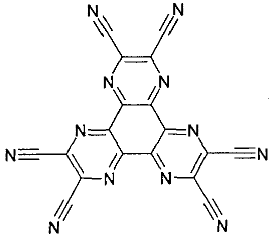 |
| HTM5 | HTM6 | HAT-CN |

| **Tabelle 2: Aufbau der OLEDs** | | | | | | | |
|---|---|---|---|---|---|---|---|
| ***Bsp.*** | ***HIL1*** | ***HTL*** | ***HIL2*** | ***EBL*** | ***EML*** | ***ETL*** | ***EIL*** |
| | *Dicke* / *nm* | *Dicke* / *nm* | *Dicke* / *nm* | *Dicke* / *nm* | *Dicke* / *nm* | *Dicke* / *nm* | *Dicke* / *nm* |
| *V1* | *HIM1: F4TCNQ(3%)* | *HIM1* | | *HTM1* | *H1:SEB1(5%)* | *ETM(50%):LiQ(50%)* | *LiQ* |
| | *20 nm* | *175 nm* | | *20 nm* | *20 nm* | *30 nm* | *1 nm* |
| *E1* | *HIM1:F4TCNQ(3%)* | *HIM1* | *HTM1:F4TCNQ(3%)* | *HTM1* | *H1:SEB1(5%)* | *ETM(50%):LiQ(50%)* | *LiQ* |
| | *20 nm* | *155 nm* | *20 nm* | *20 nm* | *20 nm* | *30 nm* | *1 nm* |
| *V2* | *HIM1:F4TCNQ(3%)* | *HIM1* | | *HTM1* | *H2:TEG(10%)* | *ETM(50%): LiQ(50%)* | *LiQ* |
| | *20 nm* | *210 nm* | | *20 nm* | *40 nm* | *30 nm* | *1 nm* |
| *E2* | *HIM1: F4TCNQ(3%)* | *HIM1* | *HTM1:F4TCNQ(3%)* | *HTM1* | *H2:TEG(10%)* | *ETM(50%):LiQ(50%)* | *LiQ* |
| | *20 nm* | *190 nm* | *20 nm* | *20 nm* | *40 nm* | *30 nm* | *1 nm* |
| *V3* | *HIM1:F4TCNQ(3%)* | *HIM1* | | *HTM2* | *H1:SEB1(5%)* | *ETM(50%):LiQ(50%)* | *LiQ* |
| | *20 nm* | *175 nm* | | *20 nm* | *20 nm* | *30 nm* | *1 nm* |
| *E3* | *HIM1:F4TCNQ(3%)* | *HIM1* | *HTM2: F4TCNQ(3%)* | *HTM2* | *H1:SEB1(5%)* | *ETM(50%):LiQ(50%)* | *LiQ* |
| | *20 nm* | *155 nm* | *20 nm* | *20 nm* | *20 nm* | *30 nm* | *1 nm* |
| *E4* | *HIM1:F4TCNQ(3%)* | *HIM1* | *HTM1:F4TCNQ(3%)* | *HTM2* | *H1:SE81(5%)* | *ETM(50%):LiQ(50%)* | *LiQ* |
| | *20 nm* | *155 nm* | *20 nm* | *20 nm* | *20 nm* | *30 nm* | *1 nm* |
| V4 | *HIM1:F4TCNQ(3%)* | *HIM1* | | *HTM3* | *H1:SEB1(5%)* | *ETM(50%):LiQ(50%)* | *LiQ* |
| | *20 nm* | *175 nm* | | *20 nm* | *20 nm* | *30 nm* | *1 nm* |
| *E5* | *HIM1:F4TCNQ(3%)* | *HIM1* | *HTM3:F4TCNQ(3%)* | *HTM3* | *H1:SEB1(5%)* | *ETM(50%):LiQ(50%)* | *LiQ* |
| | *20 nm* | *155 nm* | *20 nm* | *20 nm* | *20 nm* | *30 nm* | *1 nm* |
| E6 | *HIM1:F4TCNQ(3%)* | *HIM1* | *HTM1:F4TCNQ(3%)* | *HTM3* | *H1:SEB1(5%)* | *ETM(50%):LiQ(50%)* | *LiQ* |
| | *20 nm* | *155 nm* | *20 nm* | *20 nm* | *20 nm* | *30 nm* | *1 nm* |
| *V5* | *HIM1:F4TCNQ(3%)* | *HIM1* | | *NPB* | *H1:SEB1(5%)* | *ETM* | *LiQ* |
| | *20 nm* | *175 nm* | | *20 nm* | *20 nm* | *30 nm* | *3 nm* |
| *E7* | *HIM1:F4TCNQ(3%)* | *HIM1* | *NPB:F4TCNQ(3%)* | *NPB* | *H1:SEB1(5%)* | *ETM* | *LiQ* |
| | *20 nm* | *155 nm* | *20 nm* | *20 nm* | *20 nm* | *30 nm* | *3 nm* |
| *V6* | *HIM2:F4TCNQ(3%)* | *HIM2* | | *HTM1* | *H1:SEB2(5%)* | *ETM(50%):LiQ(50%)* | *LiQ* |
| | *10 nm* | *140 nm* | | *30 nm* | *20 nm* | *30 nm* | *1 nm* |
| *V7* | *HIM2: F4TCNQ(3%)* | | | *HTM1* | *H1:SEB2(5%)* | *ETM(50%): LiQ(50%)* | *LiQ* |
| | *150 nm* | | | *30 nm* | *20 nm* | *30 nm* | *1 nm* |
| *E8* | *HIM2:F4TCNQ(3%)* | *HIM2* | *HTM1:F4TCNQ(3%)* | *HTM1* | *H1:SE82(5%)* | *ETM(50%): LiQ(50%)* | *LiQ* |
| | *10 nm* | *140 nm* | *20 nm* | *10 nm* | *20 nm* | *30 nm* | *1 nm* |
| *V8* | *HIM1: F4TCNQ(3%)* | *HIM1* | | *HTM4* | *H1:SEB1(5%)* | *ETM(50%):LiQ(50%)* | *LiQ* |
| | *20 nm* | *160 nm* | | *20 nm* | *20 nm* | *30 nm* | *1 nm* |
| *E9* | *HIM1:F4TCNQ(3%)* | *HIM1* | *HTM5:F4TCNQ(3%)* | *HTM4* | *H1:SEB1(5%)* | *ETM(50%):LiQ(50%)* | *LiQ* |
| | *20 nm* | *140 nm* | *20 nm* | *20 nm* | *20 nm* | *30 nm* | *1 nm* |
| *V9* | *HIM9:F4TCNQ(3%)* | *HIM1* | | *HTM5* | *H1:SEB1(5%)* | *ETM(50%):LiQ(50%)* | *LiQ* |
| | *20 nm* | *175 nm* | | *20 nm* | *20 nm* | *30 nm* | *1 nm* |
| *E10* | *HIM1:F4TCNQ(3%)* | *HIM1* | *HTM6:F4TCNQ(3%)* | *HTM5* | *H1:SEB1(5%)* | *ETM(50%):LiQ(50%)* | *LiQ* |
| | *20 nm* | *155 nm* | *20 nm* | *20 nm* | *20 nm* | *30 nm* | *1 nm* |
| *E11* | *HIM1:F4TCNQ(3%)* | *HIM1* | *HTM1:F4TCNQ(3%)* | *HTM5* | *H1:SEB1(5%)* | *ETM(50%):LiQ(50%)* | *LiQ* |
| | *20 nm* | *155 nm* | *20 nm* | *20 nm* | *20 nm* | *30 nm* | *1 nm* |
| *V10* | *HIM1:F4TCNQ(3%)* | *HIM1* | | *HTM6* | *H1:SEB1(5%)* | *ETM(50%):LiQ(50%)* | *LiQ* |
| | *20 nm* | *175 nm* | | *20 nm* | *20 nm* | *30 nm* | *1 nm* |
| *E12* | *HIM1:F4TCNQ(3%)* | *HIM1* | *HTM6:F4TCNQ(3%)* | *HTM6* | *H1:SEB1(5%)* | *ETM(50%):LiQ(50%)* | *LIQ* |
| | *20 nm* | *155 nm* | *20 nm* | *20 nm* | *20 nm* | *30 nm* | *1 nm* |
| *E13* | *HIM1:F4TCNQ(3%)* | *HIM1* | *NTM1:F4TCNQ(3%)* | *HTM6* | *H1:SEB1(5%)* | *ETM(50%):LiQ(50%)* | *LiQ* |
| | *20 nm* | *155 nm* | *20 nm* | *20 nm* | *20 nm* | *30 nm* | *1 nm* |
| *V11* | *HIM2:F4TCNQ(3%)* | *HIM2* | *Hat-CN* | *HTM1* | *H1:SEB2(5%)* | *ETM(50%): LiQ(50%)* | *LiQ* |
| | *10 nm* | *140 nm* | *10 nm* | *20 nm* | *20 nm* | *30 nm* | *1 nm* |

### Beispiel 1

Es wurde eine Referenzprobe V1 hergestellt und mit der erfindungsgemäßen Probe E1 verglichen. HIM1 und HTM1 sind in diesem Beispiel dasselbe Material. Die Referenzprobe V1 hat bei einer Stromdichte von 10 mA/cm² eine Spannung von 4.0 V, eine externe Quanteneffizienz von 7.7 % und eine Lebensdauer (LD80 @ 60 mA/cm²) von 105 h. Verglichen damit ist bei der erfindungsgemäßen Probe E1 sowohl die externe Quanteneffizienz bei einer Stromdichte von 10 mA/cm² mit 8.1 % höher, als auch die gemessene Lebensdauer (LD80 @ 60 mA/cm²) von 220 h niedriger bei gleichzeitig niedrigerer Spannung von 3.9 V. Die Farbkoordinaten nach CIE 1931 sind für die Vergleichsprobe V1 (0.14/0.14) und für die erfindungsgemäße Probe E1 (0.14/0.14).

Ein weiterer Vergleich ist die Referenzprobe V2 mit der erfindungsgemäßen Probe E2. Auch hierbei ist das Material HIM1 und HTM1 identisch. Auch hier hat die erfindungsgemäße Probe E2 sowohl eine höhere Quanteneffizienz (@ 2 mA/cm²) von 20.0 % gegenüber der Referenzprobe V2 von 19.9% als auch eine höhere Lebensdauer (LD80 @ 20 mA/cm²) von 165 h gegenüber der Referenzprobe E2 von 110 h. Die Spannung der Referenzprobe (@ 2 mA) betrug 3.3 V und lag höher als die Spannung der Probe E2 mit 3.1 V. Die CIE-Farbkoordinaten der Proben waren (0.34 / 0.63).

### Beispiel 2

In diesem Beispiel sind jeweils unterschiedliche Materialien in der Lochtransportschicht A und C vorhanden.
Die erfindungsgemäßen Proben E3 und E4 zeigen gegenüber der Referenzprobe V3 eine deutlich höhere Lebensdauer (LD80 @ 60 mA/cm²) von 305 h (E3) und von 135 h (E4) gegenüber 45 h (V3). Die Quantemeffizienz (@ 10 mA/cm²) der Referenzprobe V3 ist mit 8.9 % etwas höher als die von Probe E3 mit 8.3 % und etwas niedriger als die von Probe E4 mit 9.8 %. Die Spannung der Referenzprobe war mit 4.4 V bei 10 mA/cm² höher als die der Proben E3 mit 4.1 V und E4 mit 4.2 V.

### Beispiel 3

In diesem Beispiel sind jeweils unterschiedliche Materialien in der Lochtransportschicht A und C vorhanden.
Die Referenzprobe V4 zeigt gegenüber den erfindungsgemäßen Proben E5 und E6 eine deutlich niedrigere Lebensdauer (LD80 @ 60 mA/cm²) von 75 h im Vergleich zu E5 von 175 h und E6 von 145 h. Die Spannung der beiden erfindungsgemäßen Proben ist jeweils niedriger mit 4.0 V (E5) und 3.8 V (E6) im Vergleich zur Referenz mit 4.2 V bei 10 mA/cm².

### Beispiel 4

In diesem Beispiel sind unterschiedliche Materialien in der Lochtransportschicht A und C vorhanden.
Die Referenzprobe V5 zeigt gegenüber der erfindungsgemäßen Probe E7 eine kürzere Lebensdauer (LD80 @ 60 mA/cm²) von 105 h im Vergleich zu E7 von 125 h und höhere Spannung von 3.8 V im Vergleich zu 3.6 V bei 10 mA/cm².

### Beispiel 5

In diesem Beispiel sind unterschiedliche Materialien in der Lochtransportschicht A und C vorhanden.
Die Referenzproben V6 und V7 zeigen gegenüber der erfindungsgemäßen Probe E8 eine niedrigere Lebensdauer (LD80 @ 80 mA/cm²) von 65 h (V6), bzw. 95 h (V7) im Vergleich zu E8 von 270 h und höhere Spannungen von 4.6 V (V6) und 4.1 V (V7) gegenüber E8 mit 4.0 V bei 10 mA/cm². Die CIE-Farbkoordinaten für alle 3 Proben lagen bei (0.14/0.19).
Im Vergleich dazu hat die Referenzprobe V11, welche statt der p-dotierten Zwischenschicht eine Schicht enthaltend die Verbindung HAT-CN besitzt, zwar auch sehr niedrige Spannungen von 3.8 V, aber eine niedrigere Lebensdauer (LD80 @ 80 mA/cm²) von ca. 210 h.

### Beispiel 6

In diesem Beispiel sind unterschiedliche Materialien in der Lochtransportschicht A und C vorhanden.

Die erfindungsgemäße Probe E9 zeigt gegenüber der Referenzprobe V8 eine bessere Lebensdauer (LD80 @ 60 mA/cm²) von 215 h im Vergleich zu 155 h und niedrigere Spannungen von 3.7 V im Vergleich zu 4.4 V

### Beispiel 7

In diesem Beispiel sind unterschiedliche Materialien in der Lochtransportschicht A und C vorhanden.
Die Referenzprobe V9 zeigt gegenüber den erfindungsgemäßen Proben E10 und E11 eine niedrigere Lebensdauer (LD80 @ 60 mA/cM²) von 175 h und eine niedrigere Effizienz (EQE @ 10 mA) von 9.2 % im Vergleich zu E10 von 210 h und 9.7 %, bzw. E11 mit 255 h und 9.8 % EQE. Auch hier ist die Spannung der Referenzprobe mit 4.0 V höher als die von E10 mit 3.7 V und E11 mit 3.8 V bei 10 mA/cm².

### Beispiel 8

In diesem Beispiel sind unterschiedliche Materialien in der Lochtransportschicht A und C vorhanden.
Im Vergleich zu den erfindungsgemäßen Proben E12 und E13 zeigt die Referenzprobe V10 eine kürzere Lebensdauer (LD80 @ 60 mA/cm²) von 165 h im Vergleich zu 450 h (E12) und 405 h (E13). Auch hier ist die Spannung der Referenzprobe mit 4.3 V höher als die von E12 mit 3.96 V und E13 mit 3.7 V bei 10 mA/cm².

Wie in den obenstehenden Beispielen gezeigt, weisen die erfindungsgemäßen Vorrichtungen eine höhere Effizienz auf sowie bevorzugt eine längere Lebensdauer als Vorrichtungen gemäß dem Stand der Technik. Weiterhin ist bevorzugt die Betriebsspannung der Vorrichtungen niedriger als bei Vorrichtungen gemäß dem Stand der Technik.

## Patentansprüche

1. Elektronische Vorrichtung enthaltend Anode, Kathode und mindestens eine zwischen Anode und Kathode angeordnete emittierende Schicht sowie
- mindestens eine Lochtransportschicht A, enthaltend mindestens ein Lochtransportmaterial
- mindestens eine p-dotierte Lochtransportschicht B, enthaltend mindestens einen p-Dotanden und mindestens eine Lochtransportmaterial-Matrix
- mindestens eine Lochtransportschicht C, enthaltend mindestens ein Lochtransportmaterial,
wobei die Lochtransportschichten A, B und C zwischen Anode und emittierender Schicht angeordnet sind, und
wobei die Lochtransportschicht B kathodenseitig von der Lochtransportschicht A angeordnet ist, und die Lochtransportschicht C kathodenseitig von der Lochtransportschicht B angeordnet ist, und wobei zwischen der Anode und der Lochtransportschicht A eine p-dotierte Lochtransportschicht A', enthaltend mindestens einen p-Dotanden und eine Lochtransportmaterial-Matrix, angeordnet ist, **dadurch gekennzeichnet,**
**dass** die Lochtransportschichten A', A, B und C jeweils eine oder mehrere gleiche oder verschiedene Triarylamin-Verbindungen enthalten, und die Lochtransportschichten A', A, B und C unmittelbar aneinander angrenzen.

2. Elektronische Vorrichtung nach Anspruch 1, **dadurch gekennzeichnet, dass** sie gewählt ist aus organischen lichtemittierenden Transistoren (OLETs), organischen lichtemittierenden elektrochemischen Zellen (OLECs), organischen Laserdioden (O-Laser) und organischen Elektrolumineszenzvorrichtungen (OLEDs).

3. Elektronische Vorrichtung nach Anspruch 1 oder 2, **dadurch gekennzeichnet, dass** die Anode Wolframoxid, Molybdänoxid und/oder Vanadiumoxid enthält.

4. Elektronische Vorrichtung nach einem oder mehreren der Ansprüche 1 bis 3, **dadurch gekennzeichnet, dass** die Lochtransportschicht A eine Dicke von 100 bis 300 nm aufweist.

5. Elektronische Vorrichtung nach einem oder mehreren der Ansprüche 1 bis 4, **dadurch gekennzeichnet, dass** die Lochtransportschicht A als Lochtransportmaterial dieselbe Verbindung enthält wie die Lochtransportschicht A' als Lochtransportmaterial-Matrix.

6. Elektronische Vorrichtung nach einem oder mehreren der Ansprüche 1 bis 5, **dadurch gekennzeichnet, dass** die Lochtransportschicht A keinen p-Dotanden enthält.

7. Elektronische Vorrichtung nach einem oder mehreren der Ansprüche 1 bis 6, **dadurch gekennzeichnet, dass** der p-Dotand gewählt ist aus Chinodimethanverbindungen, Azaindenofluorendionen, Azaphenalenen, Azatriphenylenen, I₂, Metallhalogeniden, Metalloxiden, Übergangsmetallkomplexen, und Übergangsmetalloxiden.

8. Elektronische Vorrichtung nach einem oder mehreren der Ansprüche 1 bis 7, **dadurch gekennzeichnet, dass** der p-Dotand in Lochtransportschicht B in einer Konzentration von 0.1 bis 20 Vol-% vorliegt.

9. Elektronische Vorrichtung nach einem oder mehreren der Ansprüche 1 bis 8, **dadurch gekennzeichnet, dass** die Lochtransportschicht C keinen p-Dotanden enthält.

10. Elektronische Vorrichtung nach einem oder mehreren der Ansprüche 1 bis 9, **dadurch gekennzeichnet, dass** die Lochtransportmaterialien der Lochtransportschichten A und C unterschiedlich sind.

11. Elektronische Vorrichtung nach einem oder mehreren der Ansprüche 1 bis 10, **dadurch gekennzeichnet, dass** die Lochtransportschicht B als Lochtransportmaterial-Matrix dieselbe Verbindung aufweist wie die Lochtransportschicht C als Lochtransportmaterial.

12. Elektronische Vorrichtung nach einem oder mehreren der Ansprüche 1 bis 11, **dadurch gekennzeichnet, dass** die Lochtransportschichten A, B, C und A' jeweils eine oder mehrere gleiche oder verschiedene Mono-Triarylaminverbindungen enthalten.

13. Elektronische Vorrichtung nach einem oder mehreren der Ansprüche 1 bis 12, **dadurch gekennzeichnet, dass** mindestens eine der Lochtransportschichten A, B, C und A' mindestens eine Verbindung gemäß einer der Formeln (I) bis (VI) enthält 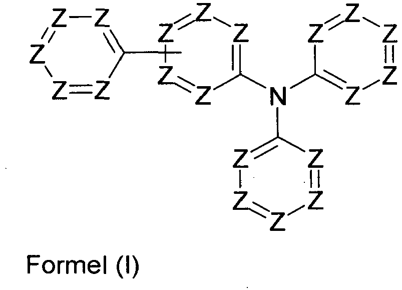 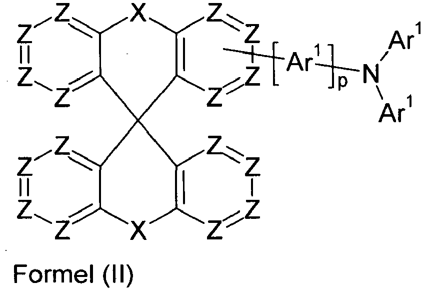 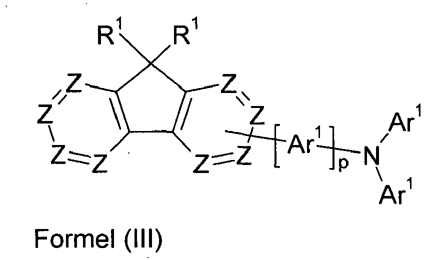 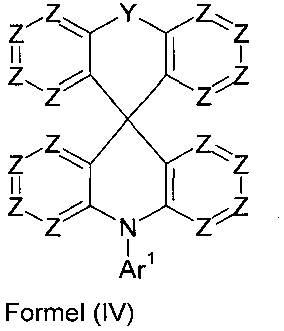 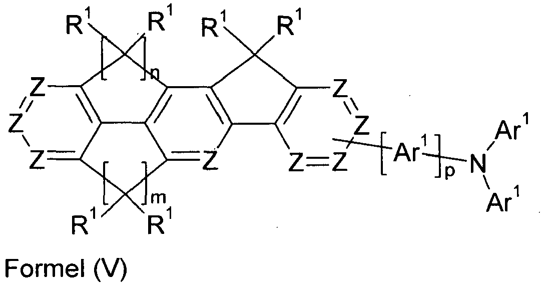 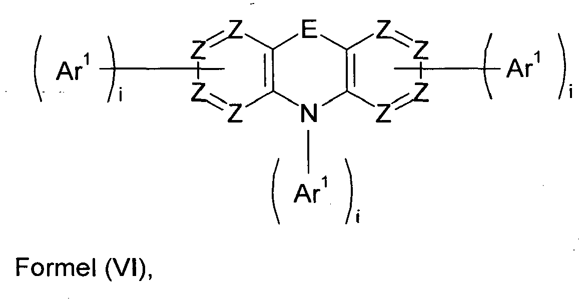 wobei gilt:
Z ist bei jedem Auftreten gleich oder verschieden N oder CR¹, wobei Z gleich C ist, wenn ein Substituent gebunden ist;
X, Y sind bei jedem Auftreten gleich oder verschieden eine Einfachbindung, O, S, Se, BR¹, C(R¹)₂, Si(R¹)₂, NR¹, PR¹, C(R¹)₂-C(R¹)₂, oder CR¹=CR¹;
E ist O, S, Se, BR¹, C(R¹)₂, Si(R¹)₂, NR¹, PR¹, C(R¹)₂-C(R¹)₂, oder CR¹=CR¹;
Ar¹ ist bei jedem Auftreten gleich oder verschieden ein aromatisches oder heteroaromatisches Ringsystem mit 5 bis 60 aromatischen Ringatomen, welches durch einen oder mehrere Reste R¹ substituiert sein kann; und
R¹ ist bei jedem Auftreten gleich oder verschieden H, D, F, Cl, Br, I, CHO, C(=O)R², P(=O)(R²)₂, S(=O)R², S(=O)₂R², CR²=CR²R², CN, NO₂, Si(R²)₃, OSO₂R² eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C≡C , Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ring-atomen, das jeweils durch einen oder mehrere Reste R² substituiert sein kann, oder eine Kombination dieser Systeme; dabei können zwei oder mehrere benachbarte Substituenten R¹ auch miteinander ein mono- oder polycyclisches, aliphatisches oder aromatisches Ringsystem bilden;
R² ist bei jedem Auftreten gleich oder verschieden H, D, CN oder ein aliphatischer, aromatischer und/oder heteroaromatischer Kohlenwasserstoffrest mit 1 bis 20 C-Atomen, in dem auch H-Atome durch D oder F ersetzt sein können; dabei können zwei oder mehrere benachbarte Substituenten R² auch miteinander ein mono- oder polycyclisches, aliphatisches oder aromatisches Ringsystem bilden;
i bei jedem Auftreten gleich oder verschieden 0 oder 1 ist, wobei die Summe aller i mindestens gleich 1 ist;
p gleich 0 oder 1 ist;
m, n gleich oder verschieden 0 oder 1 sind, wobei die Summe aus m und n gleich 1 oder 2 ist.

14. Verwendung einer elektronischen Vorrichtung nach einem oder mehreren der Ansprüche 1 bis 13 in Displays, als Lichtquelle in Beleuchtungsanwendungen sowie als Lichtquelle in medizinischen und/oder kosmetischen Anwendungen.

## Claims

1. Electronic device comprising anode, cathode and at least one emitting layer arranged between anode and cathode, and
- at least one hole-transport layer A, comprising at least one hole-transport material,
- at least one p-doped hole-transport layer B, comprising at least one p-dopant and at least one hole-transport material matrix,
- at least one hole-transport layer C, comprising at least one hole-transport material,
where the hole-transport layers A, B and C are arranged between anode and emitting layer, and
where the hole-transport layer B is arranged on the cathode side of the hole-transport layer A, and the hole-transport layer C is arranged on the cathode side of the hole-transport layer B, and
where a p-doped hole-transport layer A', comprising at least one p-dopant and a hole-transport material matrix, is arranged between the anode and the hole-transport layer A,
**characterised in that** the hole-transport layers A', A, B and C each comprise one or more identical or different triarylamine compounds, and the hole-transport layers A', A, B and C are directly adjacent to one another.

2. Electronic device according to Claim 1, **characterised in that** it is selected from organic light-emitting transistors (OLETs), organic light-emitting electrochemical cells (OLECs), organic laser diodes (O-lasers) and organic electroluminescent devices (OLEDs).

3. Electronic device according to Claim 1 or 2, **characterised in that** the anode comprises tungsten oxide, molybdenum oxide and/or vanadium oxide.

4. Electronic device according to one or more of Claims 1 to 3, **characterised in that** the hole-transport layer A has a thickness of 100 to 300 nm.

5. Electronic device according to one or more of Claims 1 to 4, **characterised in that** the hole-transport layer A comprises as hole-transport material the same compound as the hole-transport layer A' does as hole-transport material matrix.

6. Electronic device according to one or more of Claims 1 to 5, **characterised in that** the hole-transport layer A comprises no p-dopant.

7. Electronic device according to one or more of Claims 1 to 6, **characterised in that** the p-dopant is selected from quinodimethane compounds, azaindenofluorenediones, azaphenalenes, azatriphenylenes, I₂, metal halides, metal oxides, transition-metal complexes and transition-metal oxides.

8. Electronic device according to one or more of Claims 1 to 7, **characterised in that** the p-dopant is present in hole-transport layer B in a concentration of 0.1 to 20% by vol.

9. Electronic device according to one or more of Claims 1 to 8, **characterised in that** the hole-transport layer C comprises no p-dopant.

10. Electronic device according to one or more of Claims 1 to 9, **characterised in that** the hole-transport materials of the hole-transport layers A and C are different.

11. Electronic device according to one or more of Claims 1 to 10, **characterised in that** the hole-transport layer B comprises as hole-transport material the same compound matrix as the hole-transport layer C does as hole-transport material.

12. Electronic device according to one or more of Claims 1 to 11, **characterised in that** the hole-transport layers A, B, C and A' each comprise one or more identical or different monotriarylamine compounds.

13. Electronic device according to one or more of Claims 1 to 12, **characterised in that** at least one of the hole-transport layers A, B, C and A' comprises at least one compound of one of the formulae (I) to (VI) 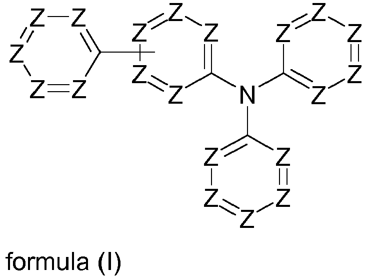 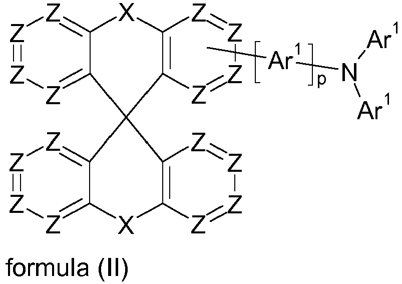 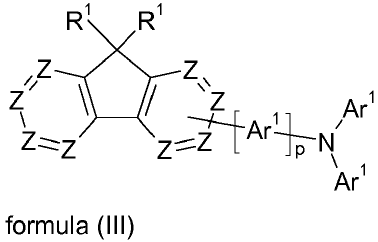 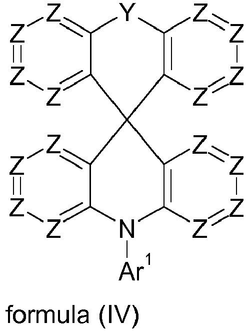 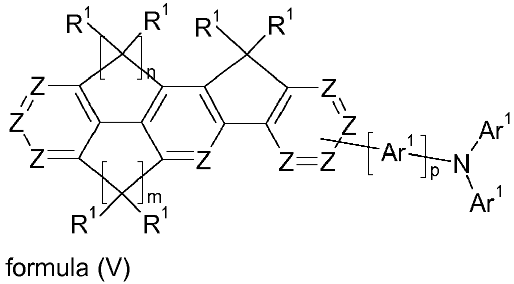 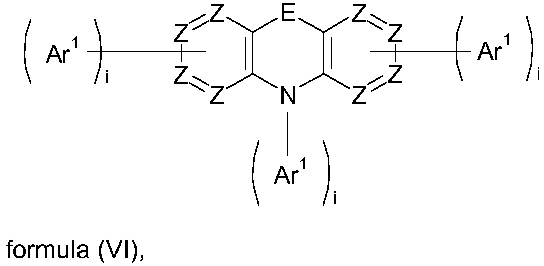 where:
Z is on each occurrence, identically or differently, N or CR¹, where Z is equal to C if a substituent is bonded;
X, Y are on each occurrence, identically or differently, a single bond, O, S, Se, BR¹, C(R¹)₂, Si(R¹)₂, NR¹, PR¹, C(R¹)₂-C(R¹)₂ or CR¹=CR¹;
E is O, S, Se, BR¹, C(R¹)₂, Si(R¹)₂, NR¹, PR¹, C(R¹)₂-C(R¹)₂ or CR¹=CR¹;
Ar¹ is on each occurrence, identically or differently, an aromatic or heteroaromatic ring system having 5 to 60 aromatic ring atoms, which may be substituted by one or more radicals R¹; and
R¹ is on each occurrence, identically or differently, H, D, F, Cl, Br, I, CHO, C(=O)R², P(=O)(R²)₂, S(=O)R², S(=O)₂R², CR²=CR²R², CN, NO₂, Si(R²)₃, OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thio-alkoxy group having 3 to 40 C atoms, which may in each case be substituted by one or more radicals R², where one or more non-adjacent CH₂ groups may be replaced by R²C=CR², C≡C, Si(R²)₂, Ge(R²)₂, Sn(R²)₂, C=O, C=S, C=Se, C=NR², P(=O)(R²), SO, SO₂, NR², O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or a combination of these systems; two or more adjacent substituents R¹ here may also form a mono- or polycyclic, aliphatic or aromatic ring system with one another;
R² is on each occurrence, identically or differently, H, D, CN or an aliphatic, aromatic and/or heteroaromatic hydrocarbon radical having 1 to 20 C atoms, in which, in addition, H atoms may be replaced by D or F; two or more adjacent substituents R² here may also form a mono- or polycyclic, aliphatic or aromatic ring system with one another;
i is on each occurrence, identically or differently, 0 or 1, where the sum of all i is at least equal to 1;
p is equal to 0 or 1;
m, n are, identically or differently, 0 or 1, where the sum of m and n is equal to 1 or 2.

14. Use of an electronic device according to one or more of Claims 1 to 13 in displays, as light source in lighting applications and as light source in medical and/or cosmetic applications.

## Revendications

1. Dispositif électronique comprenant une anode, une cathode et au moins une couche d'émission qui est agencée entre l'anode et la cathode ; et
- au moins une couche de transport de trous A, laquelle comprend au moins un matériau de transport de trous ;
- au moins une couche de transport de trous dopée selon le type p B, laquelle comprend au moins un dopant de type p et au moins une matrice de matériau(x) de transport de trous ;
- au moins une couche de transport de trous C, laquelle comprend au moins un matériau de transport de trous ;
dispositif électronique dans lequel les couches de transport de trous A, B et C sont agencées entre l'anode et la couche d'émission ; et dispositif électronique dans lequel la couche de transport de trous B est agencée sur le côté de la cathode de la couche de transport de trous A, et la couche de transport de trous C est agencée sur le côté de la cathode de la couche de transport de trous B ; et
dispositif électronique dans lequel une couche de transport de trous dopée selon le type p A', laquelle comprend au moins un dopant de type p et au moins une matrice de matériau(x) de transport de trous, est agencée entre l'anode et la couche de transport de trous A ; **caractérisé en ce que** les couches de transport de trous A', A, B et C comprennent chacune un ou plusieurs composé(s) de triarylamine identiques ou différents, et les couches de transport de trous A', A, B et C sont directement adjacentes les unes aux autres.

2. Dispositif électronique selon la revendication 1, **caractérisé en ce qu'**il est sélectionné parmi les transistors à émission de lumière organiques (OLET), les cellules électrochimiques à émission de lumière organiques (OLEC), les diodes laser organiques (O-laser) et les dispositifs électroluminescents organiques (OLED).

3. Dispositif électronique selon la revendication 1 ou 2, **caractérisé en ce que** l'anode comprend de l'oxyde de tungstène, de l'oxyde de molybdène et/ou de l'oxyde de vanadium.

4. Dispositif électronique selon une ou plusieurs des revendications 1 à 3, **caractérisé en ce que** la couche de transport de trous A présente une épaisseur de 100 à 300 nm.

5. Dispositif électronique selon une ou plusieurs des revendications 1 à 4, **caractérisé en ce que** la couche de transport de trous A comprend, en tant que matériau de transport de trous, le même composé que celui que la couche de transport de trous A' comprend en tant que matrice de matériau(x) de transport de trous.

6. Dispositif électronique selon une ou plusieurs des revendications 1 à 5, **caractérisé en ce que** la couche de transport de trous A ne comprend pas de dopant de type p.

7. Dispositif électronique selon une ou plusieurs des revendications 1 à 6, **caractérisé en ce que** le dopant de type p est sélectionné parmi les composés de quinodiméthane, les azaindénofluorènediones, les azaphénalènes, les azatriphénylènes, I₂, les halogénures de métaux, les oxydes de métaux, les complexes de métaux de transition et les oxydes de métaux de transition.

8. Dispositif électronique selon une ou plusieurs des revendications 1 à 7, **caractérisé en ce que** le dopant de type p est présent dans la couche de transport de trous B selon une concentration de 0,1 % à 20 % en volume.

9. Dispositif électronique selon une ou plusieurs des revendications 1 à 8, **caractérisé en ce que** la couche de transport de trous C ne comprend pas de dopant de type p.

10. Dispositif électronique selon une ou plusieurs des revendications 1 à 9, **caractérisé en ce que** les matériaux de transport de trous des couches de transport de trous A et C sont différents.

11. Dispositif électronique selon une ou plusieurs des revendications 1 à 10, **caractérisé en ce que** la couche de transport de trous B comprend, en tant que matériau de transport de trous, la même matrice de composé(s) que celle que la couche de transport de trous C comprend en tant que matériau de transport de trous.

12. Dispositif électronique selon une ou plusieurs des revendications 1 à 11, **caractérisé en ce que** les couches de transport de trous A, B, C et A' comprennent chacune un ou plusieurs composé(s) de monotriarylamine identiques ou différents.

13. Dispositif électronique selon une ou plusieurs des revendications 1 à 12, **caractérisé en ce qu'**au moins l'une des couches de transport de trous A, B, C et A' comprend au moins un composé de l'une des formules (I) à (VI) : 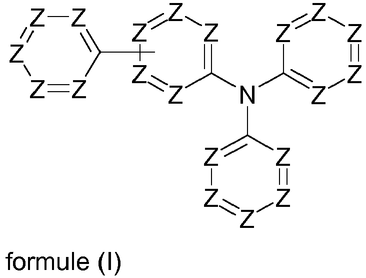 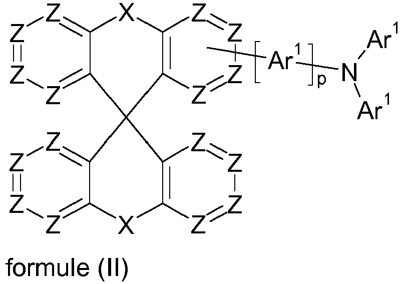 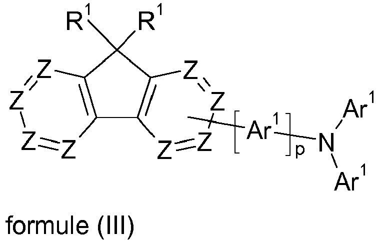 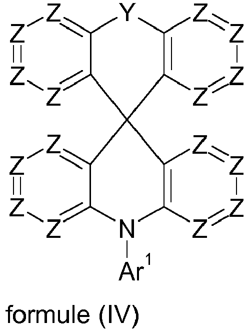 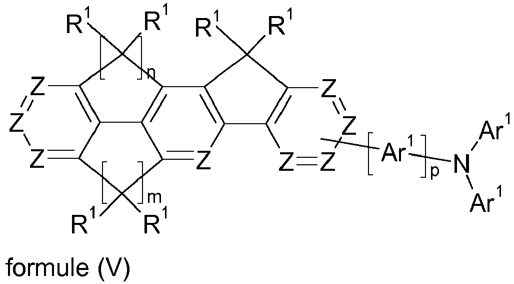 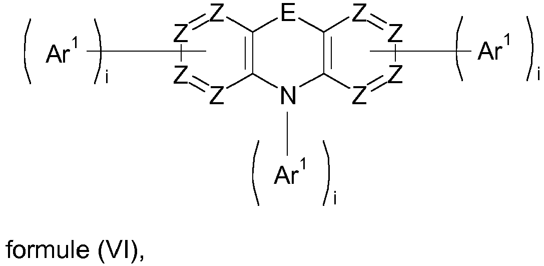 dans lesquelles :
Z est pour chaque occurrence, de manière identique ou différente, N ou CR¹, où Z est égal à C si un substituant est lié ;
X, Y sont pour chaque occurrence, de manière identique ou différente, une liaison simple, O, S, Se, BR¹, C(R¹)₂, Si(R¹)₂, NR¹, PR¹, C(R¹)₂-C(R¹)₂ ou CR¹=CR¹ ;
E est O, S, Se, BR¹, C(R¹)₂, Si(R¹)₂, NR¹, PR¹, C(R¹)₂-C(R¹)₂ ou CR¹=CR¹ ;
Ar¹ est pour chaque occurrence, de manière identique ou différente, un système de cycle aromatique ou hétéroaromatique qui comporte de 5 à 60 atomes de cycle aromatique, lequel peut être substitué par un radical ou par plusieurs radicaux R¹ ; et
R¹ est pour chaque occurrence, de manière identique ou différente, H, D, F, CI, Br, I, CHO, C(=O)R², P(=O)(R²)₂, S(=O)R², S(=O)₂R² CR²=CR²R², CN, NO₂, Si(R²)₃, OSO₂R², un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy ou thioalcoxy ramifié ou cyclique qui comporte de 3 à 40 atomes de C, lequel peut dans chaque cas être substitué par un radical ou par plusieurs radicaux R², où un ou plusieurs groupe(s) CH₂ non adjacents peut/peuvent être remplacé(s) par R²C=CR², C≡C, Si(R²)₂, Ge(R²)2, Sn(R²)₂, C=O, C=S, C=Se, C=NR², P(=O)(R²), SO, SO₂, NR², O, S ou CONR² et où un ou plusieurs atome(s) de H peut/peuvent être remplacé(s) par D, F, CI, Br, I, CN ou NO₂, ou un système de cycle aromatique ou hétéroaromatique qui comporte de 5 à 60 atomes de cycle aromatique, lequel peut dans chaque cas être substitué par un radical ou par plusieurs radicaux R², ou une combinaison de ces systèmes ; deux substituants R¹ adjacents ou plus peuvent ici également former un système de cycle aliphatique ou aromatique monocyclique ou polycyclique l'un avec l'autre ou les uns avec les autres ;
R² est pour chaque occurrence, de manière identique ou différente, H, D, CN ou un radical hydrocarbone aromatique et/ou hétéroaromatique aliphatique qui comporte de 1 à 20 atome(s) de C, où, en outre, des atomes de H peuvent être remplacés par D ou F ; deux substituants R² adjacents ou plus peuvent ici également former un système de cycle aliphatique ou aromatique monocyclique ou polycyclique l'un avec l'autre ou les uns avec les autres ;
i est pour chaque occurrence, de manière identique ou différente, 0 ou 1, où la somme de tous les i est au moins égale à 1;
p est égal à 0 ou 1;
m, n sont, de manière identique ou différente, 0 ou 1, où la somme de m et n est égale à 1 ou 2.

14. Utilisation d'un dispositif électronique selon une ou plusieurs des revendications 1 à 13 dans des affichages, en tant que source de lumière dans des applications d'éclairage et en tant que source de lumière dans des applications médicales et/ou cosmétiques.
